# FASCICULE DE BREVET EUROPEEN

(11) **EP 1 049 775 B1**
(45) Date de publication et mention de la délivrance du brevet: **07.03.2007**
(21) Numéro de dépôt: 99901671.0
(22) Date de dépôt: 29.01.1999
(51) Int. Cl.: C12N 15/12, C07K 14/47, C12N 15/85, C07K 16/18, A61K 38/17, A01K 67/027

(54) **PROTEINE HUMAINE BTRCP**
HUMANES BTRCP PROTEIN
HUMAN BTRCP PROTEIN

(30) Priorité: 30.01.1998 FR 9801100; 09.12.1998 FR 9815545
(43) Date de publication de la demande: 08.11.2000
(73) Titulaire: INSTITUT NATIONAL DE LA SANTE ET DE LA RECHERCHE MEDICALE (INSERM), 75654 Paris Cédex 13 (FR)
(72) Inventeur: BENAROUS, Richard, F-75013 Paris (FR); MARGOTTIN, Florence, F-75015 Paris (FR); DURAND, Hervé, F-91850 Bouray-sur-Juine (FR); ARENZANA SEISDEDOS, Fernando, F-92190 Meudon (FR); KROLL, Mathias, F-75015 Paris (FR); CONCORDET, Jean-Paul, F-94300 Vincennes (FR)
(74) Mandataire: Gillard, Marie-Louise
(86) Numéro de dépôt international: PCT/FR1999/000196
(87) Numéro de publication internationale: WO 1999/038969

(56) Documents cités:
- EP-A- 0 342 860
- WO-A-95/21252
- HUDSON J.W. ET AL.: "Beta-transducin repeat containing protein-3" EMBL SEQUENCE DATABASE,1 février 1997, XP002082116 HEIDELBERG DE
- MARGOTTIN F ET AL.: "Interaction between the cytoplastic domains of HIV-1 Vpu and CD4: Role of Vpu residues involved in CD4 interaction and in vitro CD4 degradation" VIROLOGY., vol. 223, 1996, pages 381-386, XP002082117 RLANDO US cité dans la demande
- DATABASE WPI Section Ch, Week 9629 Derwent Publications Ltd., London, GB; Class B04, AN 96-283507 XP002082120 & JP 08 119996 A (JAPAN TOBACCO INC) , 14 mai 1996
- BAI C ET AL.: "SKP1 connects cell cycle regulators to the ubiquitin proteolysis machinery through a novel motif, the F-box" CELL, vol. 86, no. 2, 26 juillet 1996, pages 263-274, XP002082118 NA US
- NEER E.J. ET AL.: "The ancient regulatory-protein family of WD-repeat proteins" NATURE., vol. 371, 22 septembre 1994, pages 297-300, XP002082119 LONDON GB
- PATTON E E ET AL: "Combinatorial control in ubiquitin-dependent proteolysis: don't Skp the F-box hypothesis" TRENDS IN GENETICS, vol. 14, no. 6, juin 1998, page 236-243 XP004121083
- MARGOTTIN F ET AL: "A novel human WD protein, h-beta TrCP, that interacts with HIV-1 Vpu connects CD4 to the ER degradation pathway through an F-box motif" MOLECULAR CELL, vol. 1, no. 4, mars 1998, pages 565-574, XP002101956 CAMBRIDGE US
- YARON A ET AL.: "Identification of the receptor component of the IkappaBalpha-ubiquitin ligase" NATURE., vol. 396, no. 6711, 10 décembre 1998, pages 590-594, XP002101957 LONDON GB
- LATRES E ET AL: "The human F box protein beta-TrCP associates with the Cul1/Skp1 complex and regulates the stability of beta-catenin" ONCOGENE, vol. 18, no. 4, 28 janvier 1999, pages 849-854, XP002101958
- SKOWYRA D ET AL.: "The SCF ubiquitin ligase in cell cycle control and byond" MOLECULAR BIOLOGY OF THE CELL, vol. 9, no. suppl., novembre 1998, page 240A XP002101959

## Description

La présente invention a pour objet une nouvelle protéine humaine qui intervient dans le ciblage des protéines vers les voies de dégradation par le protéasome. Cette protéine, dénommée h-βTrCP, est capable d'interagir notamment avec la protéine Vpu du virus HIV-1, ainsi qu'avec les protéines cellulaires IκB, β-caténine et Skp1p.

La dégradation des protéines par le protéasome, complexe multi-protéique présent dans toutes les cellules, est impliquée dans de nombreux phénomènes cellulaires essentiels comme le contrôle de la prolifération cellulaire, le renouvellement des protéines et l'élimination des protéines incorrectement repliées, en particulier au niveau du réticulum endoplasmique (CIECHANOVER, A., Cell, 79, 13-21, 1994). De nombreux virus, comme le virus HIV-1 qui dégrade CD4 par l'intermédiaire d'une de ses protéines Vpu (TRONO D., Cell, 82, 189-1992, 1995), utilisent à leur profit ces voies cellulaires de dégradation des protéines, dans lesquelles les protéines sont ciblées vers le protéasome par des interactions diverses avec d'autres protéines avant d'être dégradées. Pour être ciblées vers le protéasome et dégradées par celui-ci, les protéines doivent généralement être au préalable ubiquitinylées par des complexes ubiquitine-ligase. De plus, pour être ubiquitinylées, les protéines doivent souvent subir des modifications telles que des phosphorylations (CIECHANOVER, A. Embo. J., 17, 7151-7160, 1998).

A ce jour, on connaît plusieurs autres protéines de type βTrCP :
- la protéine βTrCP de Xenope décrite par Spevak et al. (Mol. Cell. Biol., 13, 4953-4966, 1993) ;
- la protéine Slimb de la drosophile décrite par Jiang et al. (Nature, vol. 391,29 Janvier 1998) ;
- la protéine KIAA0696 mise en évidence par lshikawa et al. (DNA Research, 5, 169-176, 1998) à l'occasion d'une analyse systématique de séquences exprimées dans le cerveau.

Jiang et al. ont montré que la protéine Slimb chez la drosophile est impliquée dans la stabilité de la protéine Armadillo et la signalisation de deux voies métaboliques essentielles pour le développement, à savoir les voies Hedgehog et Wingless. Ils ont également montré que la protéine Slimb a une homologie de 80% environ avec la protéine βTrCP de Xenope dont aucune fonction n'a été décrite par Spevak et al. Comme la β-caténine chez le Xenope ou chez l'homme, qui est l'homologue de la protéine Armadillo de la drosophile, semble être ciblée vers les voies de dégradation du protéasome en l'absence de signalisation des voies Hedgehog et Wingless, ils suggèrent que chez l'homme, les gènes codant pour les homologues de Slimb pourraient être impliqués dans la dégradation protéolytique de la β-caténine, protéine qui acquiert des propriétés oncogéniques quand elle n'est pas dégradée (POLAKIS, P., Biochim. Biophys. Acta 1332, F127-47, 1997).

Toutefois, même si la conservation des voies Wingless et Hedgehog chez les vertébrés est importante, il n'est pas pour autant certain que la conservation des fonctions des protéines homologues sera totale. Il y a d'ailleurs de nombreux exemples qui montrent qu'il y a toujours des différences significatives entre espèces.

De plus, Jiang et al. ont établi l'implication de Slimb dans le contrôle des voies Wingless et Hedgehog chez la drosophile uniquement sur la base d'études génétiques. Aucune preuve que ce contrôle est dépendant d'une interaction directe entre Slimb et Armadillo, par exemple, n'est ni recherchée, ni apportée.

La protéine selon l'invention, dénommée h-βTrCP, est capable d'interagir avec les protéines virales ou les protéines cellulaires susceptibles de servir de médiateurs ou d'être dégradées par le protéasome. En particulier, la protéine h-βTrCP est capable d'interagir, notamment avec la protéine Vpu du virus HIV-1, ainsi qu'avec les protéines cellulaires IκB et β-caténine.

Elle est particulièrement utile pour le criblage d'agents thérapeutiques, tels que notamment des agents antitumoraux, antiviraux, anti-inflammatoires et anti-Alzheimer.

La protéine Vpu est une petite protéine membranaire de 81 acides aminés, exprimée par la plupart des isolats du virus HIV-1 mais ni par ceux du virus humain HIV-2 nettement moins pathogène, ni par ceux du virus simien SIV (COHEN et al., Nature, 334, 532-534, 1988 ; et STREBEL et al., Science, 2, 1221-1223, 1988).

Une des fonctions de la protéine Vpu est sa capacité à induire la dégradation de la protéine CD4, récepteur cellulaire du virus HIV-1, participant ainsi à la diminution de l'expression du récepteur CD4 à la surface des cellules (Willey et al., J. Virol. 68, 1207-1212, 1994).

On sait également que les deux sérines de phosphorylation de la protéine Vpu, situées en position 52 et 56, sont indispensables pour la dégradation de CD4 induite par Vpu (MARGOTTIN et al., Virology, 223, 381-386, 1996). En outre, lors du processus d'infection par le virus HIV-1, en l'absence de la protéine Vpu, le précurseur d'enveloppe Gp160 et la protéine CD4 nouvellement synthétisée s'associent dans le réticulum endoplamique, bloquant la maturation de la protéine Gp160 (BOUR et al., J. Virol., 65, 6387-6396, 1991). La dégradation du récepteur CD4 médiée par la protéine Vpu est essentielle pour libérer la protéine d'enveloppe virale qui est retenue dans le réticulum endoplasmique par sa liaison à CD4 grâce à l'interaction avec la sous-unité Gp120, et permettre la maturation normale de l'enveloppe vers la membrane plasmique et ultérieurement son intégration dans les particules virales, ce qui les rend infectieuses. Des études récentes ont mis en évidence le fait que la dégradation du récepteur CD4 médiée par la protéine Vpu est sensible aux inhibiteurs spécifiques du protéasome et est dépendante de la présence d'une "machinerie d'ubiquitinylation intacte" ( FUJITA et al., J. Gen. Virol., 78, 619-625, 1997).

Ainsi, la protéine Vpu participe à des fonctions absolument critiques pour assurer la production de particules virales infectieuses en grand nombre, puisqu'elle intervient non seulement sur les produits du gène *gag*, c'est-à-dire sur les protéines de structure en augmentant le relâchement des particules virales, mais aussi sur ceux du gène *env* en permettant la maturation de la protéine d'enveloppe suite à la dégradation du récepteur CD4. MARGOTTIN et al. 1996 (*supra*) ont montré que l'interaction entre Vpu et CD4 se faisait par l'intermédiaire de leur domaine cytoplasmique et que cette interaction n'était pas suffisante pour déclencher la dégradation du récepteur CD4.

La protéine Skp1p est une protéine cellulaire impliquée dans le ciblage des protéines vers les voies de dégradation par le protéasome, lequel dépend de l'ubiquitinylation des protéines (PICKART C.M., The Faseb Journal, 11, 1055-1066, 1997).

BAI et al. (Cell, 86, 263-274, 1996) ont montré que la protéine Skp1p était nécessaire pour la protéolyse médiée par l'ubiquitine et que cette dégradation se faisait grâce à l'interaction de Skp1p avec des protéines contenant un motif dénommé boîte F.

La protéine Skp1p est un facteur essentiel de ciblage de protéines régulatrices du cycle cellulaire par le protéasome. Le ciblage de la dégradation de ces régulateurs est en particulier nécessaire à l'entrée du cycle cellulaire en phase S de synthèse d'ADN (PAGANO, M., The Faseb Journal, 11, 1068-1075, 1997). Des études récentes ont montré que la protéine Skp1p, avec des protéines à boite F sont les éléments essentiels de complexes de haut poids moléculaires appelés SCF pour "Skp1p-Cullin-F-box-protein complexes". Ces complexes SCF jouent le rôle d'enzyme E3 qui par leur activité ubiquitine-ligase permettent la dernière étape de l'ubiquitilylation de protéines substrats qui sont ainsi ciblées vers la dégradation par le protéasome (HOYT, A, Cell, 91, 149-151, 1997). Par ailleurs, on notera qu'il n'existe pas à ce jour d'homologue de Skp1p identifié chez la drosophile.

La protéine IκB, qui existe sous différentes formes (α, β, ε), est l'inhibiteur majeur du facteur de transcription NFκB, qui maintient celui-ci sous la forme d'un complexe inactif dans le cytoplasme (Beg A. et al. Genes and Dev., 7, 2064-2070, 1993). Après stimulation des cellules par des facteurs tels que l'interleukine 1 (IL1) et le facteur de nécrose tumorale (TNF), la protéine IκB est phosphorylée sur les résidus sérine S32 et S36. Cette phosphorylation conduit très rapidement à l'ubiquitinylation de la protéine et à son ciblage vers la dégradation par le protéasome. Le facteur NFκB actif, par exemple sous la forme de deux sous-unités P50 et P65, est alors libéré, importé dans le noyau où il va pouvoir activer de très nombreux gènes et entraîner notamment des phénomènes inflammatoires.

La protéine β-caténine est une protéine cellulaire qui contrôle les voies essentielles de transduction de signaux, comme les voies Wingless, qui sont très conservées chez tous les vertébrés. (MILLER et al., Genes and Dev. 10, 2527-2539, 1996 et POLAKIS, P., Biochim. Biophys. Acta 1332, F 127-47, 1997). La β-caténine s'accumule dans les cellules cancéreuses, soit par suite de mutations qui empêchent la phosphorylation sur les résidus sérine 33 et 37 (protéines β-caténines mutées), soit par suite de mutations de son co-facteur, la protéine APC, qui est nécessaire à sa dégradation.

L'accumulation de la β-caténine, liée à sa non-dégradation, conduit à son importation dans le noyau et à l'activation de gènes contrôlés par des promoteurs TCF-LEF, ce qui provoque des phénomènes de transformation et prolifération cellulaire.

Il a été montré récemment que des mutations de la preseniline-1 chez des patients atteints de la maladie d'Alzheimer entraînaient une déstabilisation et une dégradation accrue de la β-caténine (ZHANG et al., Nature, 395, 698-702, 1998). Ces auteurs ont montré que la preseniline-1 non mutée se lie à la β-caténine et contribue ainsi à sa stabilité. Dans la maladie d'Alzheimer, la presiniline mutée n'est plus capable de se lier à la β-caténine, et cette dernière est alors dégradée plus rapidement. Le niveau de β-caténine est nettement diminué dans les cellules neuronales de patients atteints de la maladie d'Alzheimer. La perte de la β-caténine entraîne une apoptose accrue des cellules neuronales qui serait responsable de la perte neuronale constatée dans cette pathologie.

On comprend aisément qu'il est urgent de trouver des moyens pour moduler, à savoir activer ou inhiber, le ciblage des protéines vers le protéasome.

On a maintenant trouvé une nouvelle protéine humaine qui est une protéine qui intervient dans le ciblage des protéines vers les voies de dégradation par le protéasome et qui permet de cribler des agents modulateurs du ciblage des protéines vers le protéasome.

La présente invention a donc pour objet une nouvelle protéine humaine, dénommée h-βTrCP, qui présente la SEQ ID No.2 et qui intervient dans le ciblage des protéines vers les voies de dégradation par le protéasome.

La protéine h-βTrCP possède 569 acides aminés et comporte une boîte F et sept motifs WD dont la position dans la séquence SEQ ID N° 2 est la suivante :
- boîte F : acides aminés 147-191,
- premier motif WD : acides aminés 259-292,
- deuxième motif WD : acides aminés 304-332,
- troisième motif WD : acides aminés 343-372,
- quatrième motif WD : acides aminés 387-415,
- cinquième motif WD : acides aminés 427-455,
- sixième motif WD : acides aminés 467-492,
- septième motif WD : acides aminés 516-544.

En raison de l'homologie de cette nouvelle protéine avec la βTrCP de Xenope, protéine contenant des motifs β-transducine et connue en langue anglaise sous la dénomination " beta transducin repeats containing protein", la protéine de l'invention est dénommée h-βTrCP (human-βTrCP).

La protéine h-βTrCP de l'invention est capable d'interagir, par l'intermédiaire de ses motifs WD, avec les protéines susceptibles d'être dégradées par le protéasome, en particulier avec les protéines virales et les protéines cellulaires qui possèdent le motif de phosphorylation comprenant les acides aminés Asp-Ser-Gly-Xaa-Xaa-Ser dans lequel Xaa est un acide aminé naturel quelconque et dans lequel les résidus sérine sont phosphorylés.

La phosphorylation de ce motif Asp-Ser-Gly-Xaa-Xaa-Ser est indispensable à l'ubiquitinylation et à la dégradation ultérieure des protéines qui possèdent ce type de motif. La protéine h-βTrCP n'est capable d'interagir avec les protéines contenant ce motif que lorsque les deux résidus sérine sont phosphorylés et elle ne peut interagir avec des protéines contenant un motif de phosphorylation dans lequel les résidus sérine sont mutés en acides aminés non phosphorylables. En interagissant avec les protéines phosphorylées sur ce motif, la protéine h-βTrCP contrôle leur ubiquitinylation et leur criblage vers le protéasome en vue de leur dégradation.

Parmi ces protéines, on peut citer notamment la protéine virale Vpu et les protéines cellulaires IκB et β-caténine.

On a aussi trouvé que la protéine h-βTrCP interagit, par l'intermédiaire de sa boîte F, avec la protéine Skp1p ; elle fait donc partie d'un nouveau complexe SCF, SCF-h-βTrCP, qui sélectionne certaines protéines cellulaires ou virales qui doivent être dégradées par le protéasome.

Par l'activité de ciblage vers les voies de dégradation par le protéasome, la protéine h-βTrCP, selon l'invention, joue le rôle de médiateur cellulaire de la protéine Vpu dans les cellules infectées par le virus HIV-1.

Sans pour autant vouloir se limiter à une théorie quelconque, on pense que, dans les cellules infectées par le virus HIV-1, le virus utilise, par l'intermédiaire de la protéine Vpu, le complexe SCF, dont la protéine βTrCP fait partie pour induire la dégradation du récepteur CD4 qui va favoriser la réplication virale et le relâchement des virions infectieux.

L'invention a aussi pour objet les fragments peptidiques de la protéine h-βTrCP résultant de l'addition, la suppression et/ou le remplacement d'un ou plusieurs acides aminés, lesdits fragments peptidiques ayant conservé l'activité d'interaction avec les protéines susceptibles d'être dégradées par le protéasome, en particulier avec la protéine Vpu du virus HIV-1, avec la protéine cellulaire IκB ou la protéine cellulaire β-caténine et/ou avec la protéine Skp1p.

L'invention concerne en particulier les fragments peptidiques qui comprennent au moins l'une des séquences en acides aminés de h-βTrCP ci-après :
251-569,
292-569,
292-396,
292-545 et
1-291.

On préfère tout particulièrement les fragments peptidiques dénués en partie ou en totalité de la boîte F ou ceux qui sont dénués en partie ou en totalité des motifs WD.

Un fragment peptidique particulièrement préféré est le mutant délété des résidus 32-179, dénommé ci-après βTrCPΔF.

La présente invention a également pour objet les séquences d'acides nucléiques, à savoir les séquences d'ADN génomique, les séquences d'ADNc ou d'ARNm qui comprennent ou sont constituées par un enchaînement de nucléotides codant pour la protéine h-βTrCP ou pour l'un quelconque de ses fragments peptidiques tels que définis précédemment.

L'invention concerne notamment les séquences d'acides nucléiques codant pour la protéine h-βTrCP et ses fragments peptidiques décrits ci-dessus qui sont représentées par :
a) la séquence d'ADN SEQ ID No.1 et des fragments d'acides nucléiques codant pour lesdits fragments peptidiques ;
b) les séquences d'ADN hybridant dans des conditions strictes avec la séquence a) ;
c) les séquences d'ADN qui, en raison de la dégénérescence du code génétique, résultent des séquences a) et b) ci-dessus et codent pour la protéine h-βTrCP ou ses fragments ; et
d) les séquences d'ARNm et d'ADN correspondantes.

Les protéines et fragments peptidiques selon l'invention peuvent être obtenus par la technique du génie génétique qui comprend les étapes de :
- culture d'un microorganisme transformé ou de cellules eucaryotes transformées à l'aide d'une séquence d'acides nucléiques selon l'invention et
- récupération de la protéine ou du fragment peptidique produit par ledit microorganisme ou lesdites cellules eucaryotes.

Cette technique est bien connue de l'homme du métier. Pour plus de détail la concernant, on pourra se référer à l'ouvrage ci-après : Recombinant DNA Technology 1. Editors Ales Prokop. Raskesh K Bajpai : Annals of the New-York Academy of Sciences, volume 646, 1991.

Ils peuvent également être préparés par les synthèses peptidiques classiques bien connues de l'homme du métier.

Les acides nucléiques selon l'invention peuvent être préparés par synthèse chimique et génie génétique en utilisant les techniques bien connues de l'homme du métier et décrites par exemple dans SAMBROOK et al. (*supra*).

Par exemple, la synthèse des séquences d'ADNc selon l'invention peut être effectuée par amplification des ARNm de cellules humaines à l'aide de la méthode PCR (Polymerase Chain Reaction), comme décrit par exemple par GOBLET et al. (Nucleic Acid Research, 17, 2144, 1989) en utilisant des oligonucléotides synthétiques comme amorces, définis à partir de la séquence d'ADN SEQ ID No.1.

Le fragment d'acides nucléiques amplifié peut ensuite être cloné selon les techniques décrites dans AUSUBEL et al. (Current Protocols in Molecular Biology, chapter 3, *supra*).

L'invention a également pour objet des animaux transgéniques exprimant un transgène de la protéine h-βTrCP de l'invention ou des animaux transgéniques dans lesquels le gène βTrCP a été invalidé.

Ces animaux transgéniques ou invalidés pour le gène de la protéine h-βTrCP pourront servir de modèles d'étude *in vivo* de la perturbation du cycle cellulaire et de la prolifération par l'absence ou la surexpression du gène de la protéine h-βTrCP ou de formes tronquées ou mutées de cette protéine, de la protéine Skp1p, de la protéine Vpu, de la protéine IκB ou de la protéine β-caténine.

Ces animaux transgéniques sont obtenus par des techniques bien connues de l'homme du métier, telles que celles décrites dans Manipulating the mouse embryo: a laboratory manual. HOGAN, B., BEDDINGTON, R., COSTANTINI, F. & LACY, E. Cold Spring Harbor laboratory press, second edition, 1994.

A titre d'animaux, on préfère les mammifères tels que la souris ou le rat.

L'invention a également pour objet les microorganismes procaryotes et les cellules eucaryotes transformés à l'aide d'un vecteur d'expression contenant une séquence d'ADN selon l'invention. Ce vecteur d'expression, qui peut être par exemple sous la forme d'un plasmide, doit comporter, outre la séquence d'ADN de l'invention, les moyens nécessaires à son expression, tels que notamment un promoteur, un terminateur de transcription, une origine de réplication et de préférence un marqueur de sélection. La transformation des microorganismes et des cellules eucaryotes est une technique bien connue de l'homme du métier qui pourra aisément déterminer, en fonction du microorganisme à transformer, les moyens nécessaires à l'expression de la séquence d'ADN selon l'invention.

Le microorganisme préféré aux fins de l'invention est *E. coli* alors qu'on utilise de préférence *Saccharomyces cerevisiae* comme levure.

A titre d'exemples de cellules eucaryotes qui conviennent aux fins de l'invention, on peut citer notamment les cellules COS, CHO, SF9, Jurkat, etc., toutes étant répertoriées à l'ATCC.

L'invention a également pour objet les cellules eucaryotes cotransformées avec des vecteurs d'expression contenant d'une part la séquence d'ADN codant pour la protéine Vpu, pour la protéine Skp1p, pour la protéine IκB ou pour les protéines β-caténine mutées, et d'autre part une séquence codant pour la protéine h-βTrCP, lesdits vecteurs d'expression contenant de plus des moyens utiles à leur expression, y compris dans le système double-hybride en levure.

La présente invention a donc également pour objet les agents antiviraux anti-HIV-1 qui sont constitués par les fragments peptidiques de la protéine h-βTrCP de l'invention qui ont conservé les propriétés d'interaction de la protéine h-βTrCP soit avec la protéine Vpu, soit avec la protéine Skp1p. Ces fragments peptidiques sont dénués de la boîte F ou des motifs WD de sorte qu'ils ne peuvent plus interagir avec la protéine Skp1p ou la protéine Vpu, respectivement.

On peut encore citer, à titre d'agents antiviraux, d'agents antitumoraux ou d'agents anti-inflammatoires, des anticorps spécifiques dirigés contre la protéine h-βTrCP de l'invention et ses fragments peptidiques, ce qui constitue un autre objet de l'invention.

Ces anticorps peuvent être des anticorps monoclonaux obtenus par le procédé bien connu de KOHLER et MILSTEIN (Nature, 256, 495-497, 1975) ou des anticorps polyclonaux obtenus selon les procédés classiques d'immunisation d'animaux (Antibodies, a laboratory manual. E. Harlow & D. Lane. Cold Spring Harbor laboratory press, 1988).

On peut enfin citer comme agents antiviraux, agents antitumoraux ou agents anti-inflammatoires, les oligonucléotides antisens bloquant la transcription ou la traduction de la protéine h-βTrCP de l'invention qui s'hybrident avec une séquence d'acides nucléiques telle que définie précédemment, ce qui constitue également un autre objet de la présente invention.

Ces oligonucléotides antisens sont préparés par des techniques bien connues de l'homme du métier, telles que celles décrites par AUSUBEL et al. (Current Protocols in Molecular Biology, Green Publishing Associates and Wiley-Interscience, New-York, 1989, Mises à jour jusqu'en 1997).
Les fragments peptidiques de h-βTrCP, qui possèdent la boîte F ou qui ont conservé à la fois les motifs WD et la boite F, peuvent être utilisés comme agents antitumoraux ou anti-inflammatoires.

Les fragments peptidiques de h-βTrCP, qui sont dépourvus de la boîte F, peuvent être utilisés en thérapie génique pour le traitement des maladies inflammatoires ostéo-articulaires ou des syndromes inflammatoires aiguës qui s'accompagnent d'une activation de NFκB induite par la libération massive de TNFα lors de ces processus.

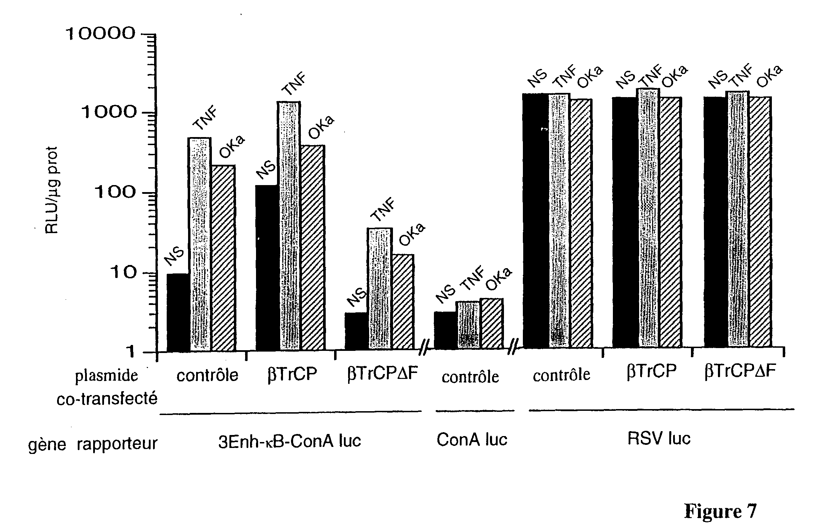

Comme illustré sur la figure 7. l'expression de h-βTrCPΔF est capable d'inhiber massivement par un facteur d'environ 20 fois l'activation transcriptionnelle induite par TNFα. Donc dans toutes les pathologies marquées par une réaction inflammatoire intense due à une libération de TNFα, la h-βTrCPΔF pourrait agir comme un puissant agent anti-inflammatoire. Il y a par exemple actuellement plusieurs tentatives pour mettre en oeuvre une thérapie génique de la polyarthrite rhumatoïde, avec injection dans les articulations lésées de virus recombinants. On peut utiliser dans ces essais de thérapie génique des syndromes inflammatoires, des vecteurs exprimant la h-βTrCPΔF. Ces vecteurs pourront être de plusieurs types (rétrovirus, adénovirus ; ANDERSON F., Nature, 392, 25-30, 1998). L'expression de la h-βTrCPΔF pourra être contrôlée par ses effets sur l'inhibition de l'activation de NFκB par TNF.

La présente invention a aussi pour objet l'utilisation de la protéine h-βTrCP ou des séquences d'acides nucléiques codant pour cette protéine ou pour ses fragments peptidiques pour le criblage d'agents thérapeutiques susceptibles de moduler l'interaction de la protéine h-βTrCP avec les protéines susceptibles d'être dégradées par le protéasome, en particulier pour le criblage :
- d'agents antiviraux anti-HIV-1 susceptibles d'inhiber l'interaction entre la protéine h-βTrCP et la protéine Vpu et/ou d'inhiber l'interaction entre la protéine h-βTrCP et la protéine Skp1p,
- d'agents antitumoraux capables de perturber la régulation du cycle cellulaire ou des processus de dégradation des protéines dans des cellules humaines tumorales par modulation (inhibition ou activation) de l'interaction entre la protéine h-βTrCP et la protéine Skp1p, ainsi que par réactivation de l'interaction entre la protéine h-βTrCP et les protéines β-caténine mutées dans les cellules tumorales ou entre la protéine βTrCP et la protéine β-caténine normale dans les cellules tumorales dépourvues de la protéine APC.
- d'agents anti-inflammatoires capables de perturber l'activation du facteur de transcription NFκB par inhibition de l'interaction entre la protéine h-βTrCP et la protéine IκB,
- d'agents anti-Alzheimer capables de réduire le taux de dégradation de la β-caténine dans les cellules neuronales par inhibition de l'interaction entre la protéine h-βTrCP et la protéine β-caténine.

En effet, en perturbant les interactions Vpu/h-βTrCP et/ou Skp1p/h-βTrCP, on peut :
- soit inhiber la réplication et la production du virus HIV-1 par des cellules infectées :

- soit inhiber l'entrée en phase S du cycle cellulaire et avoir un effet antiprolifératif.

En perturbant les interactions IκB/h-βTrCP et/ou Skp1p/h-βTrCP, on peut inhiber la dégradation de la protéine IκB par le protéasome et donc inhiber l'activation du facteur de transcription NFκB.

Enfin, en activant l'interaction β-caténine mutée/h-βTrCP, on peut activer la dégradation de la β-caténine accumulée dans les cellules tumorales. En inhibant l'interaction β-caténine /h-βTrCP chez les patients atteints de la maladie d'Alzheimer, on peut réduire l'apoptose des cellules neuronales.

### Criblage de modulateurs de l'interaction h-βTrCP/protéines

On peut sélectionner les agents antiviraux soit à partir de banques aléatoires de peptides, à la surface de phages (SCOTT J. et al., Science, 249, 386-390, 1990), soit en utilisant des oligonucléotides de synthèse aléatoires selon la technique de type SELEX (TUERK et GOLD, Science, 249, 505-510, 1990). Cette technique permet d'isoler à partir d'un pool très large d'oligonucléotides, ceux qui ont une grande affinité pour la protéine d'intérêt, dans le cas présent la protéine h-βTrCP. Ils sont dénommés aptamers. Parmi ces aptamers, on pourra sélectionner ceux qui inhibent les deux interactions Vpu/h-βTrCP et Skp1p/hβTrCP par le criblage ci-après.

Le criblage défini ci-dessus peut par exemple être réalisé en utilisant le système double-hybride en levure dans lequel des cellules de levure co-exprimant la protéine h-βTrCP selon l'invention et l'une des protéines Vpu, IκB ou β-caténine, la protéine Skp1p, sont cultivées sur des milieux sélectifs appropriés en présence de la substance à tester : les milieux sélectifs sont les milieux couramment utilisés dans ce domaine et donc bien connus de l'homme du métier.

Le système double-hybride en levure est décrit par FIELDS et SONG dans Nature, 340-245-246, 1989 et dans le brevet US 5 667 973. Ce système double-hybride, est basé sur la détection des interactions protéine-protéine par activation du gène rapporteur His ou LacZ sous le contrôle de domaines de l'activateur transcriptionnel Gal4 dans la levure.

Dans ce système double-hybride, on cotransforme une levure par un vecteur double-hybride contenant l'ADNc de l'une des protéines et un vecteur contenant l'ADNc de l'autre protéine, chacun desdits vecteurs contenant soit un domaine de liaison à l'ADN soit un domaine d'activation de la transcription. On fait ensuite exprimer par la levure les deux protéines dans un milieu de culture approprié, par exemple un milieu de culture sans histidine. L'interaction entre les deux protéines hybrides permet l'activation du gène His3 et la croissance des levures sur un milieu sans histidine d'une part, ainsi que l'activation du gène LacZ qui est révélée par une réaction colorimétrique spécifique de la β-galactosidase. On peut donc vérifier l'interaction lorsque les levures poussent sur un milieu sans histidine et lorsqu'on observe une réaction colorimétrique.

On peut également utiliser le test du halo tel que décrit par Valtz & Peter (Meth-Enzymol-283, 350-365, 1997) pour détecter s'il y a interaction.

On peut également utiliser des variantes du système double-hybride, telles que le système triple-hybride décrit par TIRODE et al. (J. Biol. Chem., 272, 22995-22999, 1997), ou par COLAS et al. (Nature, 380, 548-550, 1996) dans lequel un peptide inhibiteur de l'interaction peut être exprimé comme troisième partenaire pour inhiber l'interaction des deux autres. Une banque de peptides aléatoires peut aussi être utilisée de la sorte.

On peut aussi utiliser le système reverse-hybride décrit par VIDAL et al. (Proc. Natl. Acad. Sci., 93, 10315-10320), dans lequel on sélectionne non pas pour une interaction mais contre une interaction. On peut dans ce système, comme dans le système double-hybride classique, cribler des banques de petites molécules chimiques, y compris issues de la synthèse chimique pour mettre, les levures cotransformées avec les vecteurs double-hybride ou réverse hybride porteurs des fusions avec la protéine Vpu, la protéine IκB, la β-caténine, la protéine h-βTRCP ou la protéine Skp1p, en présence de ces petites molécules à la recherche d'un inhibiteur des interactions Vpu-h-βTrCP et Skp1p-h-βTrCP ou β-caténine-h-βTrCP ou encore IκB-h-βTrCP.

Les tests de criblage d'inhibiteurs d'interaction pourront également être effectués en double-hybride par conjugaison (FROMONT-RACINE et al., Nature Genetics, 16, 277-282, 1997), par double-hybride membranaire (BRODER Y.C. et al., Curr. Biol., 8, 1121-1124, 1998), et éventuellement, si les phosphorylations peuvent prendre place dans les bactéries, par double-hybride bactérien (KARIMOVA et al., Proc. Natl. Acad. Sci., 95, 5752-5756,1998).

Ce criblage peut aussi être effectué *in vitro* en utilisant l'une des protéines Vpu, ItcB, β-caténine, ou la protéine Skp1p, et la protéine h-βTrCP, l'une des protéines étant immobilisée sur un support approprié et l'autre étant marquée par un moyen quelconque utilisé dans les moyens de détection de substances biologiques, ce moyen de marquage pouvant être par exemple un isotope radioactif, un agent luminescent, de la biotine ou un anticorps spécifique.

L'une des protéines sera de préférence immobilisée sous la forme d'une protéine de fusion avec la gluthation S-transférase (GST) sur des billes d'agarose-gluthation ou en plaques de microtitration, la GST servant d'agent de couplage de ladite protéine sur les billes ou sur les puits des plaques.

A cet effet, on peut utiliser notamment le test de scintillation à proximité (SPA) décrit par BOSWORTH et al. (Nature, 341, 167-168, 1989) et commercialisé par Amersham. Ce test consiste à marquer par un élément radioactif, par exemple le tritium, l'une des protéines et à immobiliser l'autre protéine sur des billes magnétiques ou sur des billes d'agarose-gluthation. L'effet inhibiteur des substances à tester sur les interactions impliquant la protéine h-βTrCP peut être facilement détecté sans séparation des espèces radioactives liées ou libres selon les protocoles décrits par BOSWORTH et al. (*supra*).

On peut également utiliser la technique "Surface Plasmon Resonances" décrite par KARLSSON et al. (J. Immunol. Methods, 145, 229-233, 1991) utilisant le Biacore commercialisé par Pharmacia, pour isoler les inhibiteurs des interactions impliquant la protéine h-βTrCP selon l'invention.

L'activité inhibitrice des agents antiviraux ainsi sélectionnés pourra être vérifiée par des tests sur des cellules T CD₄+ ou sur des chimpanzés infectés par les virus HIV-1 ou SIV Cpz.

On peut également isoler les agents antitumoraux et les agents anti-inflammatoires, ligands de la protéine h-βTrCP de l'invention, par les techniques double-hybride ou apparentées ou par interaction *in vitro* avec des banques combinatoires de peptides ou autres produits chimiques, comme décrit précédemment.

La spécificité des agents antiviraux, antitumoraux ou anti-inflammatoires sélectionnés par le test double-hybride peut être ensuite déterminée par culture de cellules de mammifères, par exemple de cellules humaines transfectées avec la protéine β-TrCP ou un fragment de celle-ci, en présence d'un gène rapporteur spécifique de la protéine impliquée dans la pathologie que l'on souhaite traiter.

Ainsi, pour la protéine IκB, on pourra utiliser des cellules humaines provenant des lignées cellulaires Hela, 293, 293T, etc. et le gène rapporteur dépendant des sites NFκB (3Enh-κB-ConA Luc) qui contrôle l'expression de la luciférase.

Dans les cellules humaines non stimulées, la protéine βTrCP humaine est exprimée transitoirement à partir d'un vecteur d'expression eucaryote tel que pCDNA3 (Invitrogen) ou tout autre vecteur d'expression eucaryote, ayant inséré l'ADN codant pour la protéine βTrCP sous le contrôle d'un promoteur fort du cytomégalovirus CMV ou autre. Une quantité de l'ordre de 3µg de ce vecteur qui permet l'expression de la protéine βTrCP sera cotransfectée par l'une des techniques courantes de transfection (phosphate de calcium, lipofectamine (Life technologies), électroporation (Ausubel et Sambrook, ci-après) etc., avec 1µg d'un vecteur rapporteur dépendant (3Enh-κB-ConA luc) ou indépendant (RSV Luc ou ConA Luc) de sites NFκB qui contrôlent l'expression du gène rapporteur luciférase. Des molécules capables d'inhiber l'interaction h-βTrCP-IκB inhiberont l'augmentation d'expression de luciférase dans ce test. Ces inhibiteurs seront ajoutés au milieu de culture pendant au moins 6 heures, 24, 36 ou 48 heures après la transfection. On pourra contrôler la spécificité de ces inhibiteurs en vérifiant qu'ils n'ont aucun effet sur RSV Luc ou ConA Luc. On pourra également utiliser le système Dual luciférase de Promega, dans lequel on peut tester en même temps deux vecteurs rapporteurs différents.

Selon un protocole expérimental similaire à celui décrit ci-dessus, mais avec des cellules stimulées, on pourra vérifier que l'inhibition induite par l'expression du fragment h-βTrCPΔF sur l'activation transcriptionnelle TNF-dépendante a été annulée.

Ainsi, dans ce second test, les cellules humaines sont cotransfectées avec 1µg de vecteur rapporteur, soit 3Enh-κB-ConA Luc, soit ConA Luc ou RSV Luc, avec 3µg de pCDNA3 exprimant le fragment peptidique h-βTrCPLΔF, mutant de la βTrCP délétée de sa boite F. 24 à 48 h après la transfection, les cellules sont traitées pendant 6 h au TNF ou à l'acide okadaïque (OKA) qui sont de puissants activateurs de NFκB (BAUERLE et al., Cell, 1996, 87, 13-20). Le mutant h-βTrCPΔF a un effet inhibiteur massif sur l'expression du rapporteur luciférase, par rapport à un plasmide contrôle transfecté dans les mêmes conditions. Cet effet est dû à l'inhibition de la dégradation de IκB induite par la liaison du mutant h-βTrCPΔF en lieu et place de la protéine h-βTrCP sauvage endogène. De ce fait, un agent inhibiteur de l'interaction h-βTrCP-IκB inhibera aussi l'interaction h-βTrCPΔF-IκB, et donc inversera l'effet inhibiteur du fragment h-βTrCPΔF. Les inhibiteurs potentiels sont ajoutés au milieu dans les mêmes conditions que celles indiquées ci-dessus. On choisit dans les cellules stimulées au TNF ou à l'OKA, les inhibiteurs qui induisent une augmentation de l'expression du gène rapporteur.

Après avoir sélectionné des inhibiteurs dans les deux tests précédents, on peut vérifier dans un troisième test qu'ils sont capables d'inhiber l'activation de NFκB induite par la stimulation des cellules au TNF ou à l'OKA.

On traite avec les inhibiteurs potentiels les cellules transfectées uniquement par 1 µg de vecteur rapporteur (3Enh-κB-ConA Luc) et stimulées pendant 6 h au TNF ou à l'OKA. Ces inhibiteurs, pour être spécifiques, ne devront avoir d'effet que sur les vecteurs rapporteurs dépendants de IκB, et non sur les autres vecteurs rappporteurs (ConA ou RSV).

Pour la β-caténine, on pourra utiliser des cellules humaines provenant des lignées ci-dessus transformées avec la β-caténine mutée ou le fragment peptidique de βTrCP dépourvu de la boîte F en présence du vecteur Top-TK-Luci qui contient un multimère de sites TCF-LEF répondant à la β-caténine ou Fop-tk Luci qui contient un multimère muté inactif et qui ne répond plus à la β-caténine.

### Détection de mutations de β-catenine

De plus, comme on peut aisément distinguer une β-caténine mutée oncogénique de la β-caténine sauvage par le fait que la première, contrairement à la seconde, est incapable de se lier à la β-TrCP en double-hybride, on peut détecter des mutations de la β-caténine dans les tumeurs humaines, par mesure en double-hybride de l'interaction avec la β-TrCP.

Ce test est intéressant car on constate des mutations de la β-caténine dans de nombreux cancers, tels que cancer du colon, mélanome, hépatocarcinomes, etc. La seule manière de détecter ces mutations jusqu'à présent a été de séquencer la β-caténine à partir de RT-PCR sur l'ARN des tumeurs étudiées. Pour plus de sécurité, plusieurs séquences double brins doivent être faites dans ce test de l'art antérieur. De plus, l'existence d'une mutation ne signe pas en elle-même le caractère oncogénique de cette mutation. Il pourrait s'agir d'un polymorphisme sans aucun lien avec la tumorigénicité.

L'avantage du test double-hybride avec la protéine β-TrCP permet d'obtenir dans des délais équivalents à ceux nécessaires à l'obtention d'une séquence, une réponse claire quant au pourcentage de séquences oncogéniques mutées de la β-caténine détectées à partir de l'ARN tumoral. Sur un grand nombre de colonies, le pourcentage de formes oncogéniques de la β-caténine incapables d'interagir avec la β-TrCP, par rapport aux formes sauvages qui interagissent avec la β-TrCP. peut être déterminé précisément. Le test peut s'effectuer dans un temps équivalent à celui nécessaire pour l'obtention de quelques séquences et pour un coût réduit.

Les étapes de ce test sont les suivantes :
1- Préparation de l'ARN total d'une biopsie d'une tumeur et du tissu sain environnant comme contrôle, par l'une des diverses techniques ou kits de préparation de l'ARN (AUSUBEL et al., Current protocols in Molecular Biology).
2- Amplification par RT-PCR à partir des échantillons ARN, des séquences β-caténine de la tumeur et du tissu sain environnant, en utilisant un couple d'oligonucléotides permettant l'amplification soit uniquement de la partie N-terminale (1-130) qui contient les mutations oncogéniques les plus fréquemment rencontrées (RUBINFELD, B. et al., Science, 275, 1790-1792, 1997 ; DE LA COSTE et al., Proc. Natl.Acad.Sci. USA, 95, 8847-8851, 1998), soit de l'ensemble de la séquence codante de la β-caténine.
3- Insertion de ces fragments amplifiés par ligation dans un des vecteurs double-hybride, comme par exemple pGAD1318, de manière à obtenir une fusion en phase avec le domaine d'activation de la transcription de Gal4, ou du domaine équivalent d'activation de la transcription ou de liaison à l'ADN codé par le vecteur double-hybride utilisé.
4- Transformation de bactéries de diverses souches appropriées et étalement de la totalité de la transformation sur milieu LB-ampicilline.
5- Recueil de l'ensemble des colonies et minipréparation de plasmide (AUSUBEL, *supra*).
6- Des levures L40 ou toute autre souche de levure appropriée, seront cotransformées par le plasmide contenant les séquences β-caténine de la minipréparation ci-dessus avec un hybride de fusion contenant la βTrCP, par exemple pLexA-βTrCP, dans lequel la βTrCP est fusionnée au domaine de liaison à l'ADN de LexA. Un essai double-hybride est effectué sur l'ensemble des colonies obtenues, par exemple par étalement des levures cotransformées sur milieu DO-W-L, puis transfert des colonies sur milieu sélectif pour la détection des interactions, c'est-à-dire milieu DO-W-L-H, ou en présence de X-Gal pour la détection des interactions par production de β-galactosidase (BARTEL P. & FIELDS S.. Meth. Enzymol., 254, 241-263, 1995).

Les réactifs nécessaires pour ce test sont les suivants :
1- Un vecteur pGAD1318 prédigéré aux sites appropriés pour insérer le fragment amplifié obtenu par RT-PCR.
2- Les oligonucléotides appropriés pour amplifier la séquence de la β-caténine et insérer ensuite les séquences β-caténine amplifiées. Les oligonucléotides amorces pour l'amplification seront choisis en fonction du mode d'insertion du fragment amplifié et des sites retenus.
3- Le plasmide pBTM116-βTrCP exprimant la βTrCP en fusion avec le domaine de liaison à l'ADN de LexA.
4- A titre de contrôle des plasmides codant pour des hybrides de fusion avec des protéines contrôles, par exemple pLexRas et pGAD1318Raf.

On pourra également, pour ce test, appliquer la technique de "Gap repair" (SCHWARTZ, H., et al. Mutation detection by a two-hybrid assay., Hum. Mol. Gen., 7, 1029-1032, 1998) pour insérer la séquence du fragment amplifié de la β-caténine dans le vecteur double-hybride et transformer directement des levures sans passer par l'étape de transformation préalable dans les bactéries.

L'invention va maintenant être décrite en détail à l'aide de l'exposé expérimental ci-après.

Une grande partie des techniques décrites dans ces exemples, bien connues de l'homme du métier, est exposée en détail dans l'ouvrage de SAMBROOK et al. (*supra*) ou dans l'ouvrage de AUSUBEL et al. (*supra*).

La description ci-après sera mieux comprise à l'aide des figures 1 à 12 sur lesquelles :
- la figure 1A est la photographie d'une boîte de Petri montrant la croissance de cellules de levure cotransformées par les plasmides contenant Vpu_{c} + VBP1 ; Vpu_{c} + h-βTrCP; Vpu_{c-2/6} + h-βTrCP ; Vpu_{c} ; h-βTrCP + Vpu_{c} et h-βrCP + CD4_{c} sur milieu en présence d'histidine (His+), sur milieu en l'absence d'histidine (His-) et sur milieu en présence du substrat de la β-galactosidase X-Gal (β-Gal) :

- la figure 1B est la photographie d'un gel (Northern blot) montrant 3 ARNm de la protéine h-βTrCP de l'invention ;
- la figure 1C est la photographie d'un immunoblot montrant l'expression de la protéine h-βTrCP de l'invention ;
- la figure 2 donne les séquences de 4 protéines, h-βTrCP de l'invention et βTrCP1 de *Xenopus,* Met30p de *Saccharomyces cervisiae* et Scon2p de *Neurospora crassa* ;
- la figure 3 est la photographie d'un gel SDS-PAGE 15% montrant l'interaction entre Vpu_{c} et la protéine h-βTrCP de l'invention produite *in vitro ;*
- la figure 4 est la photographie d'une boîte de Petri montrant la croissance de cellules de levure cotransformées par les plasmides contenant Skp1p + h-βTrCP ; Skp1p +h-βTrCP-Δ7W ; Skp1p + VBP1 et Skp1p + CD4_{c} sur milieu en présence d'histidine (His+), sur milieu en l'absence d'histidine (His-) et sur milieu en présence du substrat de la β-galactosidase X-Gal (β-Gal) ;
- la figure 5 est une représentation schématique de la dégradation du récepteur CD4 induite par la protéine Vpu présentant le réseau d'interactions décrit précédemment ;
- la figure 6 est la photographie d'une boîte de Petri montrant la croissance de cellules de levure cotransformées par les plasmides contenant βTrCP + IκBα ; β-TrCP + IκBα S32-36AA ; βTrCP + Raf ; Ras + IκBα ; βTrCP + Vpuc et Ras + Raf sur milieu His+. sur milieu His- et l'expression de β-Gal ;
- la figure 7 est une représentation graphique montrant l'expression de la luciférase (exprimée en unités de lumière par µg : RLU/µg de protéine) dans les cellules transfectées avec les constructions h-βTrCP et h-βTrCPΔF et les plasmides de contrôle et les vecteurs rapporteurs ci-après : 3Enh-KB-ConA luc ; ConA luc ; RSV luc ;
- la figure 8 est la photographie d'un immunoblot montrant la détection de la protéine IκB phosphorylée ou non phosphorylée et la protéine h-βTrCP ou le fragment h-β-TrCPΔF. en présence d'anticorps anti-IκBα, anti-IκBα-S3^{Ⓟ} et anti-Myc ;
- la figure 9 est la photographie d'un immunoblot montrant la détection de la protéine IκB phosphorylée ou non phosphorylée avec la protéine h-βTrCP ou le fragment h-βTrCPΔF, en présence d'anticorps anti-IκBα-S32^{Ⓟ} et anti-IκBα ;
- la figure 10 est la photographie d'une boîte de Petri montrant la croissance de cellules de levure cotransformées par les plasmides contenant βTrCP + βCat_{1→130} ; βTrCP + βCat_{1→130} S33-37AA ; βTrCP-2 + βCat_{1→130} ; βTrCP-2 + βCat_{1→130} S33-37AA et βTrCP + βCat sur milieu His+, sur milieu His- et l'expression β-Gal ;
- la figure 11 est une représention graphique donnant l'expression de la luciférase (RLU/µg protéine) dans les cellules transfectées avec le plasmide pcDNA3, les plasmides contenant β-Cat ΔN, βTrCP, β-TrCPΔF, KIA 696 (β-TrCP-2) et KIA 696 ΔF (β-TrCP2ΔF) ;
- la figure 12 est la photographie d'un immunoblot montrant l'étude de la stabilité de la protéine β-caténine détectée avec des anticorps anti-βCat sous l'influence de l'expression de la protéine h-βTrCP ou du fragment h-βTrCPΔF détectés par l'anticorps anti-Myc.

### Exemple 1 : Criblage double-hybride en levure / mise en évidence de la séquence d'ADNc de la protéine h-βTrCP et de la protéine h-βTrCP

On a choisi comme cible le domaine cytoplasmique de la protéine Vpu. On a procédé à la fusion des résidus d'acides aminés 28 à 81 de la protéine Vpu de l'isolat LAI de HIV-1 avec le domaine de fixation de l'ADN de Gal4 (Gal4BD). La banque d'ADNc criblée était celle des cellules Jurkat (lignée lymphocytaire T humaine, ATCC n°TIB 152) et elle a été fusionnée au domaine d'activation de Gal4 Gal4AD) dans le vecteur pGAD1318 (BENICHOU et al. J. Biol. Chem., 269, 30073-30076, 1994).

Le clone de 1,3 kb qui a été isolé initialement par le système double-hybride (dénommé VBP1) code pour un ADN complémentaire partiel. Cet ADNc partiel code pour un fragment de 319 acides aminés correspondant au domaine C-terminal de la protéine h-βTrCP. Il contient sept motifs répétitifs suivis d'une queue C-terminale de 24 acides aminés. Ces motifs répétitifs, qui sont connus, sont dénommés motifs WD parce que leur extrémité se termine habituellement par la séquence Trp-Asp (WD). On notera que les motifs WD sont impliqués dans des interactions protéine-protéine (NEER et al., Nature, 371, 297-300, 1994).

Le clone ainsi isolé a été caractérisé par séquençage d'ADN sur séquenceur automatisé Applied Biosystem connu sous la dénomination ABI 373A. La technique de séquençage d'ADN est bien connue de l'homme du métier et est décrite notamment dans l'ouvrage SAMBROOK et al., "Molecular Cloning : a Laboratory Manual" Ed. Cold Spring Harbor Press, NY, 1989.

Une recherche dans les banques d'ADNc a montré que ce clone est l'homologue d'une séquence codant pour la protéine βTrCP de Xenope identifiée préalablement par SPEVAK et al. (Mol. Cell. Biol., 13, 4953-4966, 1993).

L'ADNc complet (2,1 kb) de la protéine h-βTrCP, qui a la SEQ ID No. 1, a été obtenu par la technique de polymérisation en chaîne (PCR) sur une préparation de plasmide correspondant à la banque d'ADN complémentaires de cellules Jurkat, telles que définies précédemment, dans le vecteur pGAD1318.

En plus des sept motifs WD identifiés dans le fragment C-terminal, la protéine h-βTrCP entière selon l'invention possède un domaine N-terminal d'environ 250 acides aminés. Le fragment N-terminal contient un motif pour lequel un consensus a été récemment défini sous le terme de boîte F et dont le rôle serait de cibler des protéines vers la machinerie de dégradation des protéines médiée par l'ubiquitine grâce à l'interaction de protéines contenant cette boîte F avec la protéine Skp1p (BAI et al., 1996, *supra*).

Ainsi la protéine h-βTrCP, par ses motifs WD, est d'une part capable d'interagir avec la protéine Vpu, et d'autre part possède un motif boite F qui interagit avec la protéine Skp1p et est donc capable de cibler les protéines vers les voies de dégradation par le protéasome.

La protéine h-βTrCP possède 569 acides aminés et comprend une boite F et sept motifs WD dont la position dans la séquence SEQ ID N° 2 est la suivante :
- boîte F : acides aminés 147-191,
- premier motif WD : acides aminés 259-292,
- deuxième motif WD : acides aminés 304-332,
- troisième motif WD : acides aminés 343-372,
- quatrième motif WD : acides aminés 387-415,
- cinquième motif WD : acides aminés 427-455,
- sixième motif WD : acides aminés 467-492,
- septième motif WD : acides aminés 516-544.

On a recherché si la protéine ainsi isolée avait une quelconque homologie avec des protéines déjà connues en utilisant la technique, bien connue de l'homme du métier, d'alignement des séquences selon le programme de MACAW (SCHULER et al., Proteins : structure, function and genetics, 9, 180-190, 1991).

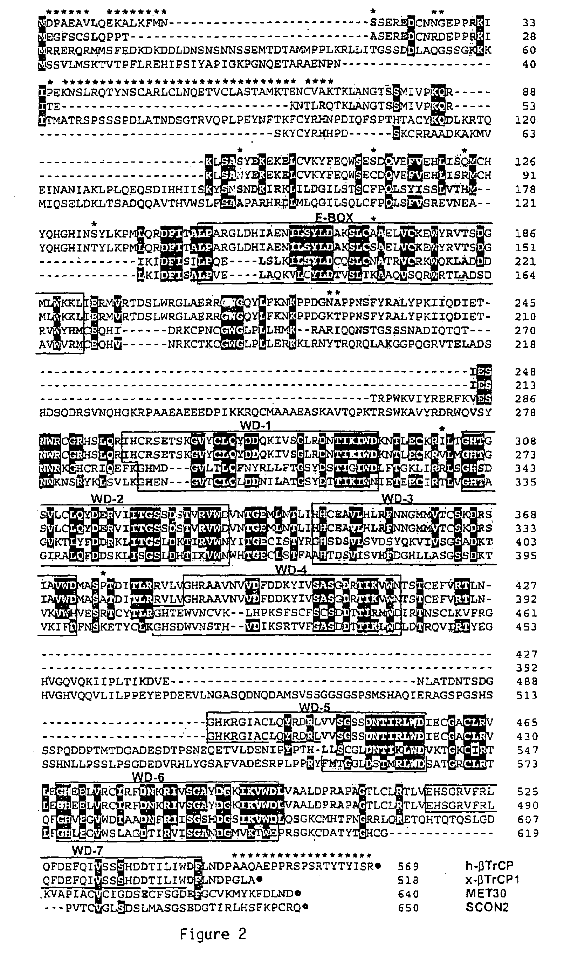

Les résultats obtenus sont reportés sur la figure 2 qui montre que la protéine h-βTrCP a une homologie :
- de 88% avec la protéine x-βTrCP1 de *Xenopus,*
- de 33% avec la protéine Met30p de *Saccharomyces cerevisiae,* inhibiteur de transcription impliqué dans la biosynthèse,
- de 31% avec la protéine Scon2p de *Neurospora crassa.*

La figure 2 montre également l'emplacement de la boîte F et des motifs WD.

### Exemple 2 : Clonage de l'ADNc de la protéine h-βTrCP

L'ADNc de la protéine h-βTrCP ayant la SEQ ID N° 1 a été amplifié par PCR à partir de 2 µg d'ADN du plasmide de la banque d'ADNc pGAD en utilisant deux tours d'amplification, le couple externe d'amorces pour le premier tour étant constitué de l'amorce sens A ayant la SEQ ID N° 3 (dans pGAD1318) et de l'amorce antisens B ayant la SEQ ID N° 4 (dans VPB1) et le couple interne d'amorces pour le deuxième tour étant constitué de l'amorce sens C ayant la SEQ ID N° 5 (dans pGAD1318) et de l'amorce antisens D ayant la SEO ID N° 6 (dans VPB1).

A la suite de cette procédure, on a isolé un fragment de 1,4 kb, souscloné dans le plasmide pGAD-VBP1 sous la forme d'un fragment 5'Spel-3'BglII, pour reconstituer le clone pGAD-h-βTrCP.

Les séquences codant pour VBP1 (résidus d'acides aminés 251 à 569 de la protéine h-βTrCP) ou codant pour la protéine h-βTrCP entière ont été sousclonées dans les vecteurs pGBT9, pGEX4T2 (Pharmacia) ou pCDNA3 (uniquement pour la protéine h-βTrCP) (Invitrogen) en utilisant des procédures standards.

### Exemple 3 : Interaction spécifique de la protéine Vpu avec la protéine h-βTrCP

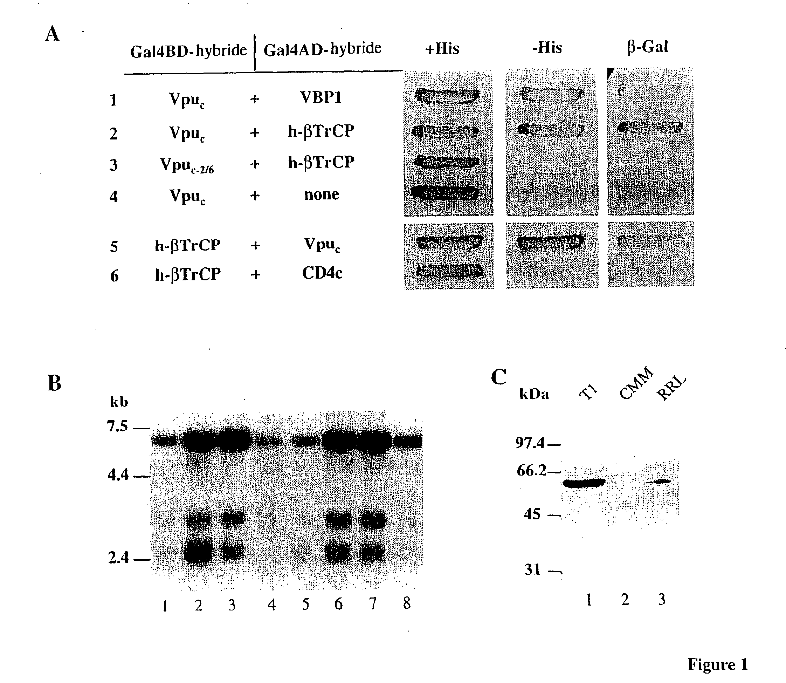

Les résultats expérimentaux qui démontrent l'interaction spécifique de la nouvelle protéine humaine βTrCP avec la protéine Vpu sont illustrés sur la figure 1.

### 3a- Interaction entre la protéine Vpu et la protéine h-βTrCP par le crible double-hybride décrit précédemment.

La figure 1A démontre l'interaction, par la technique double-hybride, de la région C-terminale de la protéine h-βTrCP (VBP1) issue de la banque d'ADNc de cellules Jurkat (ligne 1) ou de la protéine h-βTrCP entière (ligne 2) fusionnée au domaine d'activation de Gal4, avec le domaine cytoplasmique de Vpu fusionné au domaine de liaison à l'ADN de Gal4 ou vice-versa (ligne 5). L'interaction se manifeste par l'activation des deux gènes rapporteurs His3 et LacZ ; le gène His3 permet la pousse des levures en l'absence d'histidine (panneau -His), et le gène LacZ induit la production de β-galactosidase manifestée par la coloration bleue en présence du substrat X-Gal (panneau β-Gal). Cette interaction est spécifique puisqu'elle n'est pas retrouvée entre la protéine Vpu et le vecteur seul (ligne 4), ou entre la protéine h-βTrCP et une autre protéine telle la région cytoplasmique de CD4 (ligne 6). Le panneau + His est un panneau contrôle qui montre que toutes les combinaisons, y compris lorsqu'il n'y a pas d'interaction, poussent en présence d'histidine.

Il faut noter que la protéine h-βTrCP n'interagit pas avec un mutant de la protéine Vpu inactif, Vpuc-2/6 (ligne 3), clone muté sur les deux résidus sérine Ser 52 et Ser 56, qui sont essentiels pour l'activité de Vpu (MARGOTTIN et al, 1996, *supra*). Ce résultat démontre qu'il y a corrélation entre la capacité de Vpu à interagir avec la h-βTrCP et son activité.

### 3b- Mise en évidence de l'expression de la protéine h-βTrCP par analyse "Northern Blot".

Par analyse "Northem Blot" d'ARNm de différentes lignées cellulaires humaines en utilisant une sonde 5', on a trouvé que plusieurs ARN messagers (ARNm) hybrident avec une sonde correspondant à h-βTrCP (Fig. 1B). Ces ARNm de tailles respectives 2,4 kb, 3,5 kb et 7 kb, sont retrouvés dans tous les tissus humains testés. Cette multiplicité d'ARNm est réminiscente de la situation décrite par HUDSON et al. (Dev. Genet., 19, 190-198, 1996) pour les ARNm de la βTrCP de Xenope, pour laquelle 3 ARNm différents de tailles respectives voisines de celles trouvées ici pour les ARNm de la h-βTrCP ont été rapportés.

### 3c- Mise en évidence de l'expression de la protéine h-βTrCP par analyse "Immunoblot".

Des anticorps anti-peptidiques anti-h-βTrCP (Abs) ont été produits chez des lapins par immunisation avec le peptide de synthèse 275-293 correspondant au premier motif WD de la protéine h-βTrCP. Ces anticorps Abs ont été purifiés par affinité par adsorption sur 30 µg de la protéine de fusion GST-VBP1, exprimée chez *E. Coli* à partir du vecteur pGEX-VBP1 et immobilisée après électrobuvardage sur une membrane de nitrocellulose. Les anticorps Abs purifiés ont ensuite été élués par l'éluant glycine.HCl, pH 3,0, neutralisés avec du tampon 1M TRIS, pH 8,0, et utilisés pour une analyse, par la technique de Western blot, de l'expression de la protéine h-βTrCP dans les cellules humaines Sup T1 (T1), dans les réticulocytes de lapins (RRL) et dans les lysats de membrane microsomique canine (CMM).

La Figure 1C montre l'expression de la protéine h-βTrCP détectée dans un lysat de cellules T humaines de la lignée Sup T1 (ligne 1), de réticulocytes de lapin de Promega (ligne 3), par la technique "Western blot" en utilisant les anticorps anti-h-βTrCP dirigés contre le peptide 275-293 obtenus précédemment. En revanche aucune protéine correspondant à la h-βTrCP n'a pu être détectée dans des membranes de microsomes de pancréas de chien de Promega (ligne 2). La taille de la protéine h-βTrCP détectée ( 60 kD) indique que le clone d'ADNc de h-βTrCP qui a été caractérisé et qui est représenté sur la figure 2 est capable de coder pour la protéine h-βTrCP entière.

### Exemple 4 : Cartographie des sites d'interaction entre Vpuc et la protéine h-βTrCP

Les sites d'interaction entre le domaine cytoplasmique de la protéine Vpu (Vpuc) et la protéine h-βTrCP de l'invention ont été déterminés de la façon suivante.

Au niveau de Vpuc, il a été montré que la mutation des sérines aux positions 52 et 56 (clone Vpuc-2/6) abolissait intégralement l'interaction entre Vpu et h-βTrCP.

Au niveau de h-βTrCP, les résultats d'interaction double-hybride avec le domaine cytoplasmique de Vpu et les différents mutants décrits ci-après montrent que l'ensemble des motifs WD et la queue C-terminale sont requis pour une interaction optimale, comme indiqué dans le tableau ci-après.

Les mutants utilisés sont les suivants :
- VPB1-ΔW₁ (clone VPB1 dont le premier domaine WD a été délété; résidus 292 à 569), qui correspondent à un fragment BgIII-Xhol de VBP1,
- VPB1-ΔW₄₋₇ (clone VPB1 dont les domaines WD 4 à 7 ont été délétés ; résidus 292 à 396), et
- VPB1-ΔC-ter (clone VPB1 dont la queue C-terminale après le 7^{ème} domaine WD a été délétée ; résidus 292 à 545)
par PCR en utilisant respectivement l'amorce sens C, décrite précédemment, et les amorces antisens E et F suivantes dans VBP1.
Amorce E : SEQ ID N° 7
Amorce F : SEQ IND N° 8.

Le mutant h-βTrCP-Δ7W (clone h-βTrCP dont les sept domaines WD ont été délétés ; résidus 1 à 291) a été construit en insérant un fragment Spel-BglII à partir de la protéine h-βTrCP dans le vecteur pGAD1318 et le mutant βTrCPΔF (résidus délétés : 32 à 179) a été obtenu par délétion du fragment AvrII-Asp718 de la protéine h-βTrCP avec conservation du cadre de lecture.

On a vérifié que l'interaction entre les protéines Vpu et h-βTrCP pouvait avoir lieu *in vitro* par le procédé suivant : les deux protéines ont été introduites dans du lysat de réticulocytes de lapins (RRL). Les complexes Vpu/βTrCP formés *in vitro* ont été identifiés par co-immunoprécipitation en utilisant des anticorps anti-h-βTrCP dirigés contre le peptide 553-569, préparés par le même procédé que celui utilisé pour obtenir les anticorps anti-h-βTrCP dirigés contre le peptide 275-293.

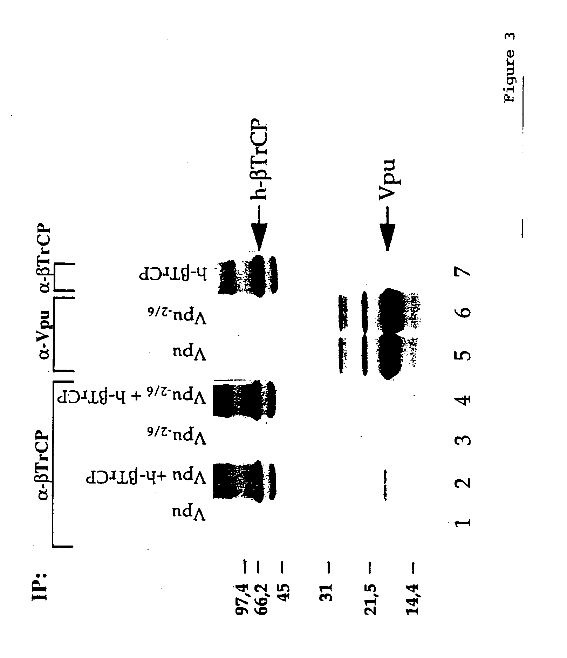

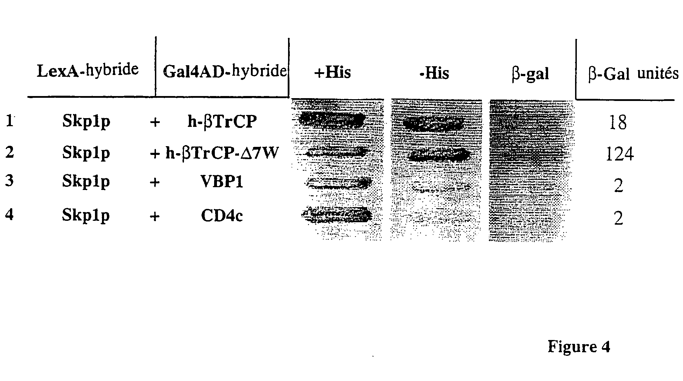

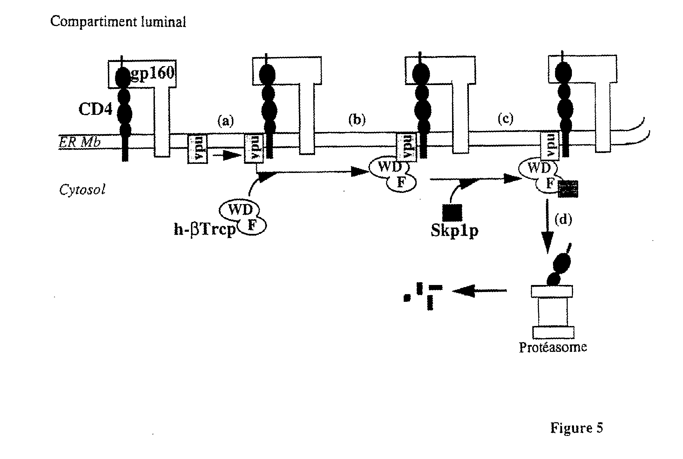

La figure 3 illustre l'interaction *in vitro* entre les protéines Vpu et h-βTrCP. La ligne 1 montre que la protéine Vpu n'est pas reconnue par l'antisérum anti-h-βTrCP, tandis que la ligne 5 montre qu'elle précipite en présence d'un antisérum anti-Vpu. La ligne 2 montre que les anticorps anti-h-βTrCP sont capables de coprécipiter la protéine Vpu co-traduite *in vitro* avec la protéine h-βTrCP. La ligne 4 montre que le double mutant de Vpu muté sur les positions Ser52 et Ser56, incapable d'induire la dégradation de CD4, n'interagit pas avec la protéine h-βTrCP, et n'est donc pas coprécipité par des anticorps anti-h-βTrCP, tandis que les lignes 6 et 7 montrent que ce mutant Vpu_{c-2/6} est traduit avec la même efficacité que la protéine Vpu.

**TABLEAU**

| Mutants à délétion de h- βTrCP | | Interaction avec Vpuc |
|---|---|---|
| h-βTrCP: | 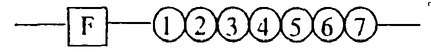 | +++ |
| VBPI: | 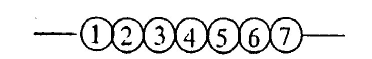 | +++ |
| VBPI-ΔWI: | 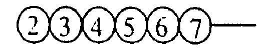 | |
| VBPI-ΔC-ter: | 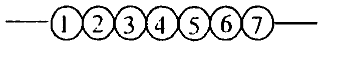 | + |
| VBP1-ΔWA-7 : | 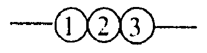 | + |
| h-βTiCP-Δ 7W : | 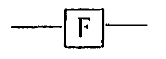 | - |

### Exemple 5 : Interaction entre la protéine h-βTrCP et la protéine Skp1p

Afin de démontrer que le motif boîte F était bien fonctionnel et pouvait donc effectivement servir au ciblage vers le protéasome par l'intermédiaire de la protéine Skp1p, on a réalisé un test double-hybride entre le domaine N-terminal de la protéine h-βTrCP et la protéine Skp1p, ce qui a permis de mettre en évidence une interaction entre la protéine h-βTrCP et la protéine Skp1p.

La protéine humaine Skp1p décrite dans BAI et al. (1996, *supra*) a été sousclonée dans le vecteur pLex10 pour une analyse d'interaction avec la protéine h-βTrCP dans la souche de levure L40 (VOJTEK et al. Cell, 74, 205-214, 1993).

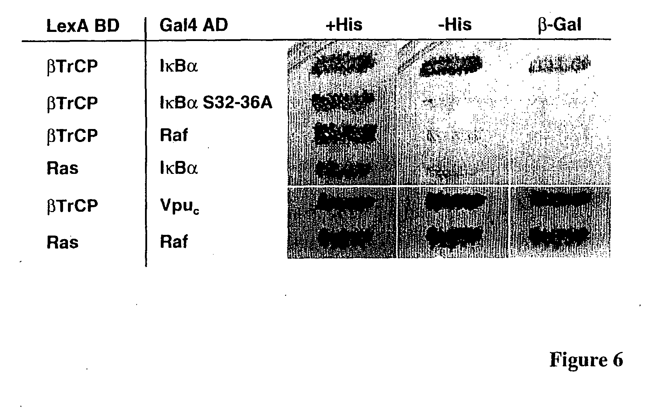

La figure 4 illustre les résultats obtenus. La ligne 1 de la figure 4 montre tout d'abord que la protéine h-βTrCP interagit avec la protéine Skp1p. La ligne 2 montre que le domaine N-terminal suffit à obtenir l'interaction, alors que la ligne 3 montre que l'absence du domaine N-terminal de la protéine h-βTrCP dans VBP1 fait perdre toute interaction avec la protéine Skp1p. Ces résultats sont des arguments supplémentaires importants en faveur d'un rôle de la protéine h-βTrCP dans la dégradation médiée par la protéine Vpu du récepteur CD4, et également corroborent les résultats de FUJITA et al. (1997, *supra*) et de SCHUBERT et al. (1997, *supra*) montrant que la dégradation du récepteur CD4 induite par la protéine Vpu devrait avoir lieu dans le protéasome. Il est à noter que le domaine cytoplasmique de CD4 est incapable de se lier directement à la protéine Skp1 (ligne 4).

### Exemple 6 : Modèle du réseau d'interactions impliqué dans la dégradation du récepteur CD4

La dégradation du récepteur CD4 induite par la protéine Vpu est effectuée par le réseau d'interactions a) entre la protéine Vpu et le récepteur CD4, b) entre la protéine Vpu et les motifs WD de la protéine h-βTrCP et c) entre la boite F de la protéine h-βTrCP et la protéine Skp1p, cette dernière interaction permettant d) le ciblage du complexe Vpu/CD4 vers le protéasome.

Ce réseau d'interactions est illustré schématiquement sur la figure 5.

C'est par l'intermédiaire d'un tel réseau d'interactions que la dégradation du récepteur CD4 par le protéasome via la protéine Vpu est provoquée.

La dégradation du récepteur CD4 permet la libération de la protéine d'enveloppe Gp160 et donc la libération du virus HIV-1 infectieux.

Un des moyens pour empêcher le développement du virus HIV-1 chez le patient atteint consiste donc à empêcher la dégradation du récepteur CD4. Un des moyens pour empêcher cette dégradation au vu du procédé de dégradation ci-dessus consiste à rechercher des inhibiteurs, ou agents antiviraux anti-HIV, inhibant l'interaction soit entre la protéine Vpu et la protéine h-βTrCP, soit entre la protéine h-βTrCP et la protéine Skp1p par les procédés décrits précédemment.

### Exemple 7 : Interaction entre la protéine h-βTrCP et la protéine IκB

Pour ce test double-hybride en levure, on a fusionné les protéines décrites ci-dessous, soit au domaine d'activation de la transcription de Gal4 (Gal4D), soit au domaine de liaison de l'ADN de LexA :
- βTrCP = protéine humaine βTrCP de la présente invention,
- IκBα,
- IκBα S32-36A = mutant de IκBα au niveau des sérines S32 et S36 de sorte qu'il n'y a pas phosphorylation,
- Ras = protéine contrôle,
- Raf = protéine contrôle,
- Vpuc = protéine Vpu cytoplasmique telle que décrite précédemment.

Les résultats expérimentaux qui démontrent l'interaction spécifique de la nouvelle protéine humaine βTrCP avec la protéine IκB sont illustrés sur la figure 6.

Grâce à ce test double-hybride, il est montré que :
- les deux protéines h-βTrCP et IκB sont capables d'interagir,
- l'interaction h-βTrCP/IκB est spécifique des deux hybrides puisque, lorsque l'un des deux hybrides est remplacé par un hybride avec une autre protéine telle que Gal4AD-Raf ou LexABD-Ras, il n'y a plus d'interaction, alors que ces deux hybrides contrôles sont capables d'interagir, et
- cette interaction est perdue quand les résidus sérines S32 et S36 de la protéine IκB sont mutés en résidus non phosphorylables comme Alanine.

Dans ce test, on a également observé une interaction entre la protéine VPu du virus HIV-1 et la protéine h-βTrCP.

### Exemple 8 : Interaction IκB/h-βTrCP en cellules humaines: modulation de l'activation transcriptionnelle de gènes rapporteurs de l'activité NFκB par l'expression de la protéine h-βTrCP ou de son fragment h-βTrCPΔF

Dans des cellules non stimulées (NS) de la lignée cellulaire 293, la protéine βTrCP humaine ou le fragment h-βTrCPΔF sont exprimés transitoirement à partir d'un vecteur d'expression eucaryote tel que pCDNA3 (Invitrogen), ayant inséré l'ADNc codant pour la protéine h-βTrCP sous le contrôle d'un promoteur du cytomégalovirus fort (CMV). Une quantité de 3 *µ*g de ce plasmide, qui permet l'expression de la protéine h-βTrCP ou du fragment h-βTrCPΔF, est cotransfectée par lipofectamine (Life technologies), avec 1 *µ*g d'un vecteur rapporteur dépendant (3Enh-κB-ConA Luc) ou indépendant (RSV Luc ou ConA Luc) de sites NFκB qui contrôlent l'expression du gène rapporteur luciférase.

Les résultats obtenus (Figure 7) montrent que le fragment h-βTrCPΔF est capable d'agir comme un transdominant négatif. Le fragment h-βTrCPΔF inhibe par compétition avec la βTrCP endogène, l'activition de NFκB induite par le TNF ou l'acide okadaique (OKA). Cette activation de NFκB est mesurée par l'activité d'un gène rapporteur sous le contrôle d'un promoteur qui a trois sites de fixation à NFκB (3Enh-κB-ConA Luc) (Arenzana et al., 1993, J. Virol., 67, 6596-6609). En revanche, la protéine h-βTrCP a un effet activateur sur l'activation de NFκB. La protéine h-βTrCP ou le fragment h-βTrCPΔF n'ont aucun effet sur la transcription d'un gène rapporteur dirigé par un promoteur ne contenant pas de sites NFκB (RSV Luc) (Invitrogen).

### Exemple 9 : Utilisation d'anticorps spécifiques pour la mise en évidence de l'interaction entre la protéine h-βTrCP et la protéine IκB endogène des cellules 293 ou Hela, et de ses conséquences sur la stabilité de la protéine IκB

La stabilité des formes phosphorylées d'IκB a été analysée dans des cellules 293 transfectées par un plasmide contrôle, par un plasmide pcDNA (Invitrogen) exprimant la protéine h-βTrCP ou par un plasmide pcDNA exprimant le fragment h-βTrCPΔF, la protéine h-βTrCP et le fragment h-βTrCPΔF ayant été fusionnés à l'épitope myc en C-terminal de ce vecteur pcDNA. Après 36 heures, on a stimulé les cellules 293 avec du TNF en présence de 100 *µ*g/ml de cycloheximide (inhibiteur de la synthèse protéique). On a séparé les protéines cytoplasmiques en gel polyacrylamide dénaturant au SDS, puis on les a transférées sur une membrane de nitrocellulose et incubées, soit avec l'anticorps monoclonal 10B dirigé contre la partie amino-terminale d'IκB et reconnaissant toutes les formes de la protéine (α-IκBα ; JAFFRAY et al., Mol. Cell. Biol., 15, 2166-2172, 1995), soit avec un anticorps polyclonal reconnaissant spécifiquement les formes phosphorylées d'IκB (α-IκBα-S32^{Ⓟ} ; 9241S, New England Biolabs), soit avec un anticorps monoclonal anti-myc dirigé contre l'épitope myc fusionné à h-βTrCP et h-βTrCPΔF et montrant l'expression de ces derniers dans les cellules transfectées (α-Myc ; SC40AC, Santa Cruz), en réalisant un Western blot (WB).

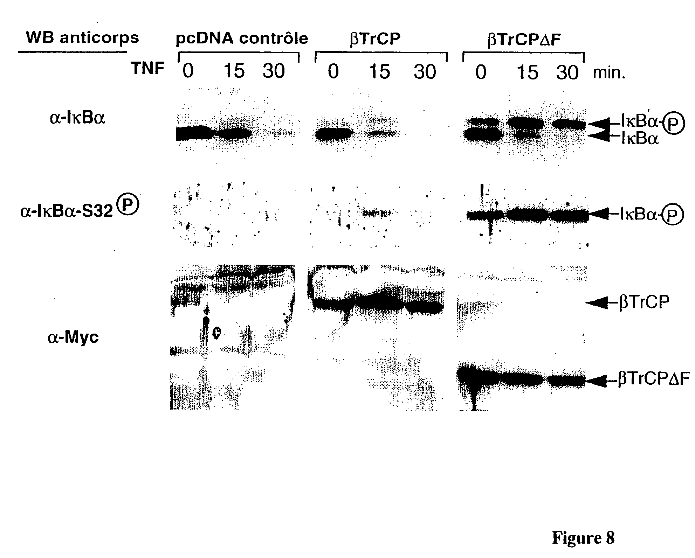

Les résultats obtenus (figure 8) montrent que l'expression du mutant h-βTrCPΔF s'accompagne de l'inhibition de la dégradation de IκB normalement induite par le TNF. Sous l'influence de l'expression de h-βTrCPΔF, les formes phosphorylées s'accumulent, comme montré par la réactivité des anticorps α-IκBα-S32^{Ⓟ}(panneau de droite).

La protéine h-βTrCP, au contraire, active la dégradation de IκBα (panneau du milieu). Le panneau inférieur concernant l'anticorps α-Myc est un panneau témoin qui montre l'expression des protéines h-βTrCP et h-βTrCPΔF.

On a également confirmé l'interaction h-βTrCP-IκB par une expérience d'immunoprécipitation.

Pour ce faire, on a transfecté des cellules Hela avec un plasmide pcDNA contrôle exprimant la β-galactosidase (β-Gal), la protéine h-βTrCP (βTrCP) ou le mutant h-βTrCPΔF (βTrCPΔF), h-βTrCP et h-βTrCPΔF ayant été fusionnés avec un épitope myc en C-terminal. Après 36 heures, on a stimulé les cellules Hela pendant 15 minutes par le TNF en présence d'inhibiteurs du protéasome (z-LLL-H) (PALOMBELLA, V. et al., Cell, 78, 773-789, 1994) (z-LLL-H + TNF ; réaction +), ou laissées sans stimulation (réaction -). On a ensuite procédé soit à un immunoblot direct après séparation des protéines du lysat cellulaire par gel dénaturant SDS et transfert sur une membrane de nitrocellulose incubée avec des anticorps α-IκBα-S32^{Ⓟ}(panneau supérieur), soit à une immunoprécipitation avec des anticorps α-Myc suivie d'un immunoblot comme indiqué ci-dessus avec l'anticorps α-IκBα-S32^{Ⓟ} ou l'anticorps α-IκBα .

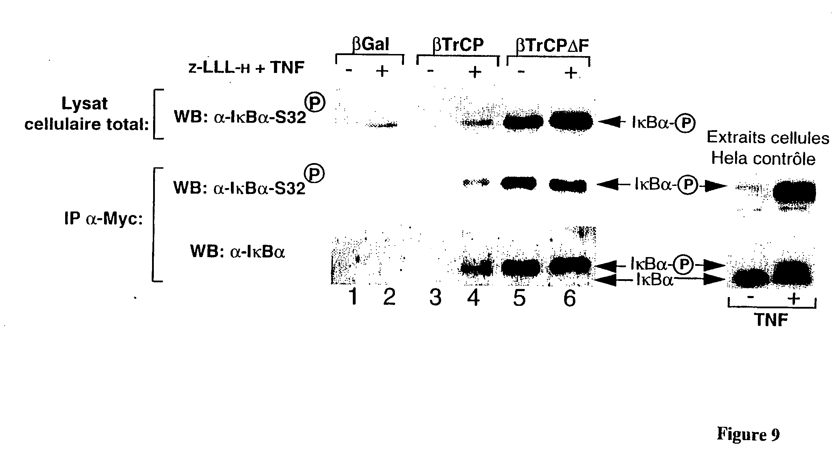

Les résultats sont indiqués sur la figure 9, sur laquelle le panneau supérieur reprend les résultats uniquement du Western blot et les deux panneaux inférieurs ceux de l'immunoprécipitation-Western blot, et le panneau de droite donne le patron de migration des formes phosphorylées induites par le traitement au TNF sur des cellules Hela contrôles.

La figure 9 montre, par des expériences de co-immunoprécipitation de la protéine h-βTrCP ou du fragment h-βTrCPΔF fusionnés avec l'épitope myc, que seule la forme phosphorylée de IκBα et non la forme non phosphorylée, est associée à la protéine h-βTrCP (colonne 4). Cette association est surtout révélée grâce à l'inhibition de la dégradation induite par le mutant h-βTrCPΔF (colonnes 5 et 6), et à l'utilisation d'inhibiteurs du protéasome (z-LLL-H).

### Exemple 10 : Interaction entre la protéine h-βTrCP et la protéine β-caténine

Pour ce test double-hybride, on a fusionné les ADNc codant pour les protéines décrites ci-dessous, soit au domaine d'activation de la transcription de Gal4 (Gal4AD), soit au domaine de liaison à l'ADN de LexA (LexABD) :
- βTrCP = protéine humaine βTrCP de la présente invention.
- KIAA0696 (βTrCP-2) = protéine humaine βTrCP isolée par ISHIKAWA et al. (DNA Research, 5, 169-176, 1998),
- βCat₁→130 = protéine β-caténine normale (domaine N-terminal ; 1→130),
- βCat_{1→130} S33-37AA = protéine β-caténine oncogène mutée sur les résidus sérine S33 et S37 de sorte qu'il n'y a pas phosphorylation,
- βCat = protéine β-caténine normale entière,

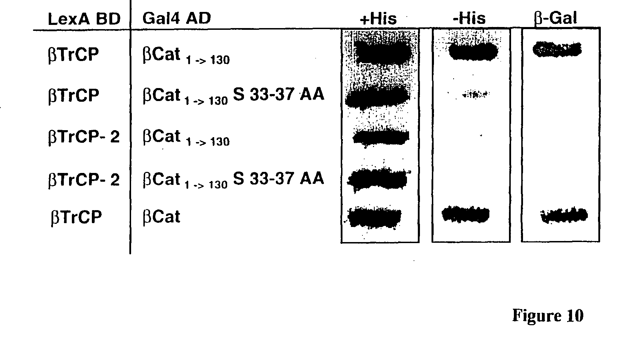

Les résultats expérimentaux qui démontrent qu'il existe une interaction spécifique de la nouvelle protéine humaine βTrCP avec la protéine β-caténine sont illustrés sur la figure 10.

Grâce à ce test double-hybride, il est montré que :
- les deux protéines h-βTrCP et βCat_{1→130} sont capables d'interagir,
- l'interaction h-βTrCP/βCat_{1→13} est perdue quand les résidus sérine S33 et S37 sont mutés en résidus non phosphorylables (β-caténine oncogène), et
- la βTrCP-2 n'est pas capable de réagir ni avec la β-caténine non mutée, ni avec la β-caténine mutée.

Il est à noter qu'on observe également une interaction, entre la protéine β-caténine entière et la protéine h-βTrCP.

### Exemple 11 : Activation de la transcription du gène rapporteur TCF/LEF par expression de la β-caténine mutée ou de h-βTrCP ΔF dans les cellules humaines 293.

On a transfecté des cellules HEK 293 avec le vecteur rapporteur Top-TK-Luci, qui contient un multimère de sites TCF-LEF ou le vecteur rapporteur Fop-TK-Luci, qui contient un multimère contrôle inactif de sites TCF-LEF. Ces constructions sont cotransfectées avec le vecteur d'expression pCDNA3 (Invitrogen), soit vide comme contrôle, soit exprimant un fragment oncogénique de la β-caténine, à savoir la β-caténine dépourvue de la partie N-terminale β-catΔN, soit exprimant la protéine h-βTrCP ou le fragment βTrCPΔF. L'activité luciférase est mesurée 24 h après la transfection et normalisée à une activité Renilla Luciférase contrôle obtenue par co-transfection de cellules avec le vecteur RSV-Renilla (Promega).

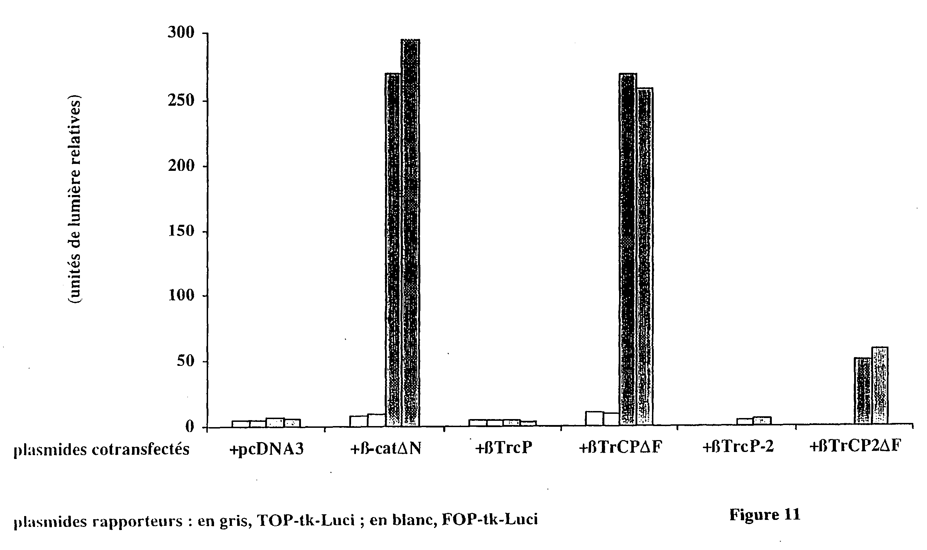

Les résultats obtenus, qui figurent sur la figure 11, montrent que le fragment h-βTrCPΔF induit une activation d'un gène rapporteur contrôlé par un promoteur TCF/LEF, qui répond à des modifications de niveau d'expression de la β-caténine (Morin P.J. et al. 1997, Science, 275, 1787-1790). Ceci indique que la dégradation de la β-caténine est inhibée par expression du mutant h-βTrCPΔF. Par contre, en ce qui concerne la protéine KIAA 0696 β-TrCP2 dans le même système de gène rapporteur, l'effet positif induit par le KIAA 0696 ΔF (βTrCP2ΔF) est beaucoup plus faible que celui obtenu avec la construction équivalente βTrCPΔF.

L'ensemble de ces résultats démontrent donc que c'est la protéine h-βTrCP de l'invention, et non la protéine KIAA 0696, qui est le médiateur de la dégradation de la β-caténine.

### Exemple 12 : Etude de l'expression de la protéine h-βTrCP ou du fragment h-βTrCPΔF sur la stabilité de la β-caténine endogène des cellules Hela

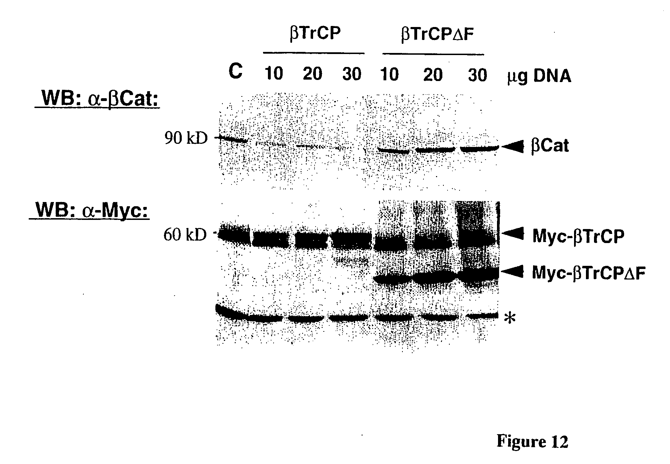

On a transfecté des cellules Hela avec les quantités d'ADN indiquées sur la figure 12, exprimant soit la protéine h-βTrCP, soit le fragment h-βTrCPΔF fusionnés à l'épitope myc en C-terminal dans un vecteur pcDNA (Invitrogen). Après 24 heures, on a lysé les cellules et on a séparé les protéines cellulaires sur gel de polyacrylamide dénaturant SDS. on les a transférées sur membrane de nitrocellulose et on les a incubées soit avec un anticorps anti-β-caténine (α-βCat), soit avec un anticorps anti-myc pour la détection de l'expression de la protéine h-βTrCP ou du fragment h-βTrCPΔF (α-Mvc) en réalisant un Western blot (WB).

Les résultats sont indiqués sur la figure 12 et montrent que l'expression de la h-βTrCP augmente, la dégradation de la β-caténine (colonne du milieu), alors que l'expression du mutant h-βTrCPΔF inhibe la dégradation de la β-caténine et conduit à son accumulation dans les cellules (colonne de droite).

Il est à noter que dans la colonne C, on montre un contrôle de cellules Hela non transfectées ; et l'astérisque indique, par le marquage non spécifique d'une protéine cellulaire du lysat de cellules Hela que, approximativement, la même quantité de protéines cellulaires a été déposée dans toutes les pistes.

Les résultats corroborent ceux montrés dans l'exemple précédent.

## Revendications

1. Protéine humaine βTrCP (h-βTrCP) de ciblage des protéines vers les voies de dégradation par le protéasome, **caractérisée en ce qu'**elle a la SEQ ID No.2, **en ce qu'**elle est capable d'interagir avec les protéines susceptibles d'être dégradées par le protéasome et qui possèdent le motif de phosphorylation comprenant les acides aminés Asp-Ser-Glu-Xaa-Xaa-Ser dans lequel Xaa est un acide aminé naturel quelconque et dans lequel les résidus sérine sont phosphorylés, et **en ce qu'**elle possède les motifs WD ci-après :
- premier motif WD : acides aminés 259-292,
- deuxième motif WD : acides aminés 304-332,
- troisième motif WD : acides aminés 343-372,
- quatrième motif WD: acides aminés 387-415,
- cinquième motif WD : acides aminés 427-455,
- sixième motif WD : acides aminés 467-492,
- septième motif WD : acides aminés 516-544.

2. Protéine selon la revendication 1, **caractérisée en ce qu'**elle est capable d'interagir avec la protéine Vpu du virus HIV-1 ou avec les protéines cellulaires IκB ou β-caténine.

3. Protéine selon la revendication 1, **caractérisée en ce qu'**elle possède une boîte F et **en ce qu'**elle est capable d'interagir avec la protéine Skp1p.

4. Protéine selon l'une quelconque des revendications 1 à 3, **caractérisée en ce que** la boîte F est constituée par les acides aminés 147-191.

5. Fragments peptidiques de la protéine selon l'une quelconque des revendications 1 à 4 résultant de l'addition, la suppression et/ou le remplacement d'un ou plusieurs acides aminés, lesdits fragments peptidiques ayant conservé l'activité d'interaction avec la protéine Vpu du virus HIV-1, la protéine cellulaire IκB ou la protéine cellulaire β-caténine et/ou avec la protéine Skp1p.

6. Séquences d'acides nucléiques codant pour la protéine humaine h-βTrCP et les fragments peptidiques selon l'une quelconque des revendications 1 à 5, **caractérisées en ce qu'**elles sont constituées par :
a) la séquence d'ADN SEQ ID No.1 et des fragments d'acides nucléiques codant pour lesdits fragments peptidiques ;
b) les séquences d'ADN hybridant dans des conditions strictes avec la séquence a) ;
c) les séquences d'ADN qui, en raison de la dégénérescence du code génétique, résultent des séquences a) et b) ci-dessus et codent pour la protéine humaine h-βTrCP ou les fragments de celle-ci ; et
d) les séquences d'ARNm et d'ADN correspondantes.

7. Utilisation de la protéine h-βTrCP ou des fragments peptidiques selon l'une quelconque des revendications 1 à 5 pour le criblage d'agents antiviraux anti-HIV-1 susceptibles d'inhiber l'interaction entre la protéine h-βTrCP et la protéine Vpu.

8. Utilisation de la protéine h-βTrCP ou des fragments peptidiques selon l'une quelconque des revendications 1 à 5 pour le criblage d'agents antiviraux anti-HIV-1 susceptibles d'inhiber l'interaction entre la protéine h-βTrCP et la protéine Skp1p.

9. Utilisation des séquences d'acides nucléiques selon la revendication 6 pour le criblage d'agents antiviraux anti-HIV susceptibles d'inhiber l'interaction entre la protéine h-βTrCP et la protéine Vpu.

10. Utilisation des séquences d'acides nucléiques selon la revendication 6 pour le criblage d'agents antiviraux anti-HIV susceptibles d'inhiber l'interaction entre la protéine h-βTrCP et la protéine Skp1p.

11. Utilisation de la protéine h-βTrCP ou des fragments peptidiques selon l'une quelconque des revendications 1 à 5 pour le criblage d'agents antitumoraux capables de perturber la régulation du cycle cellulaire ou des processus de dégradation des protéines dans des cellules humaines tumorales par modulation de l'interaction entre la protéine h-βTrCP et la protéine Skp1p.

12. Utilisation des séquences d'acides nucléiques selon la revendication 6, pour le criblage d'agents antitumoraux capables de perturber la régulation du cycle cellulaire ou des processus de dégradation des protéines dans des cellules humaines tumorales par modulation de l'interaction entre la protéine h-βTrCP et la protéine Skp 1p.

13. Utilisation de la protéine h-βTrCP ou des fragments peptidiques selon l'une quelconque des revendications 1 à 5 pour le criblage d'agents anti-inflammatoires capables de perturber l'activation du facteur de transcription NFκB par inhibition de l'interaction entre la protéine h-βTrCP et la protéine IκB.

14. Utilisation des séquences d'acides nucléiques selon la revendication 6 pour le criblage d'agents anti-inflammatoires capables de perturber l'activation du facteur de transcription NFκB par inhibition de l'interaction entre la protéine h-βTrCP et la protéine IκB.

15. Utilisation de la protéine h-βTrCP ou des fragments peptidiques selon l'une quelconque des revendications 1 à 5 pour le criblage d'agents antitumoraux susceptibles de réactiver l'interaction entre la protéine h-βTrCP et une protéine β-caténine mutée de cellules tumorales, ou entre la h-βTrCP et la β-caténine normale de cellules tumorales dépourvues de la protéine APC.

16. Utilisation des séquences d'acides nucléiques selon la revendication 6 pour le criblage d'agents antitumoraux susceptibles de réactiver l'interaction entre la protéine h-βTrCP et la protéine β-caténine mutée de cellules tumorales ou entre la h-βTrCP et la β-caténine normale de cellules tumorales dépourvues de la protéine APC.

17. Utilisation de la protéine h-βTrCP ou des fragments peptidiques selon l'une quelconque des revendications 1 à 5 pour le criblage d'agents anti-Alzheimer susceptibles de réduire le taux de dégradation de la β-caténine par inhibition de l'interaction entre la protéine h-βTrCP et la protéine β-caténine.

18. Utilisation des séquences d'acides nucléiques selon la revendication 6 pour le criblage d'agents anti-Alzheimer susceptibles de réduire le taux de dégradation de la β-caténine par inhibition de l'interaction entre la protéine h-βTrCP et la protéine β-caténine.

19. Utilisation de la protéine h-βTrCP ou des fragments peptidiques selon l'une quelconque des revendications 1 à 5 pour la détection par criblage en double-hybride en levure des mutations de la β-caténine.

20. Utilisation des séquences d'acides nucléiques selon la revendication 6 pour la détection par criblage en double-hybride en levure des mutations de la β-caténine.

21. Agents antiviraux anti-HIV qui sont constitués par les fragments peptidiques de la protéine h-βTrCP selon la revendication 5 dénués de la boîte F.

22. Agents antiviraux anti-HIV qui sont constitués par les fragments peptidiques de la protéine h-βTrCP selon la revendication 5 dénués des motifs WD.

23. Anticorps spécifiques dirigés contre la protéine h-βTrCP ou les fragments peptidiques tels que définis dans l'une quelconque des revendications 1 à 5.

24. Oligonucléotides antisens bloquant la transcription ou la traduction de la protéine h-βTrCP selon l'une quelconque des revendications 1 à 4 qui s'hybrident avec une séquence d'acides nucléiques selon la revendication 6.

25. Agents antitumoraux qui sont constitués par les fragments peptidiques de la protéine h-βTrCP selon la revendication 5, qui possèdent la boîte F.

26. Agents antitumoraux qui sont constitués par les fragments peptidiques de la protéine h-βTrCP selon la revendication 5 et qui ont conservé à la fois les motifs WD et la boite F.

27. Agents anti-inflammatoires qui sont constitués par les fragments peptidiques de la protéine h-βTrCP selon la revendication 5 dénués de la boîte F.

28. Souris transgéniques exprimant un transgène de la protéine h-βTrCP selon l'une quelconque des revendications 1 à 4.

29. Souris transgéniques dans lesquels le gène βTrCP a été invalidé.

30. Vecteur d'expression, **caractérisé en ce qu'**il comprend une séquence d'acides nucléiques selon la revendication 6 et les moyens nécessaires à son expression.

31. Microorganismes ou cellules hôtes transformés par un vecteur d'expression selon la revendication 30.

32. Microorganismes ou cellules hôtes co-transformés par un vecteur d'expression contenant le gène codant pour la protéine Vpu et un vecteur d'expression selon la revendication 30.

33. Microorganismes ou cellules hôtes cotransformés par un vecteur d'expression contenant le gène codant pour la protéine Skp1p et un vecteur d'expression selon la revendication 30.

34. Microorganismes ou cellules hôtes cotransformés par un vecteur d'expression contenant le gène codant pour la protéine IκB et un vecteur d'expression selon la revendication 30.

35. Microorganismes ou cellules hôtes cotransformés par un vecteur d'expression contenant le gène codant pour la protéine β-caténine oncogène et un vecteur d'expression selon la revendication 30.

## Claims

1. Human βTrCP protein (h-βTrCP) for the targeting of proteins towards proteasome degradation pathways, **characterized in that** it has SEQ ID No. 2 and it is capable of interacting with proteins degradable by proteasome and which possess the phosphorylation unit comprising the amino acids Asp-Ser-Glu-Xaa-Xaa-Ser in which Xaa is any natural amino acid and the serine residues are phosphorylated and **in that** it comprises the following WD units:
- first WD unit: amino acids 259-292,
- second WD unit: amino acids 304-332,
- third WD unit: amino acids 343-372,
- fourth WD unit: amino acids 387-415,
- fifth WD unit: amino acids 427-455,
- sixth WD unit: amino acids 467-492,
- seventh WD unit: amino acids 516-544.

2. Protein according to claim 1, **characterized in that** it is capable of interacting with the Vpu protein of HIV-1 virus or with the cell proteins Iκβ or β-catenin.

3. Protein according to claim 1, **characterized in that** it has a F-box and it is capable of interacting with the Skp1p protein.

4. Protein according to any one of claims 1 to 3, **characterized in that** the F-box is constituted by the amino acids 147-191.

5. Peptide fragments of the protein according to any one of claims 1 to 4 which result from the addition, deletion and/or replacement of one or more amino acids, said peptide fragments having conserved the activity of interacting with the Vpu protein of HIV-1 virus, the cell protein IκB or the cell protein β-catenin and/or with the Skp1p protein.

6. Nucleic acid sequences coding for the human protein h-βTrCP or peptide fragments according to any one of claims 1 to 5, **characterized in that** they consist of:
a) the DNA sequence SEQ ID No. 1 and the DNA sequences of the nucleic acid fragments coding for said peptide fragments;
b) the DNA sequences which hybridise under strict conditions with the sequence a);
c) the DNA sequences which, due to the degeneracy of the genetic code, result from the sequences a) and b) above and code for the human protein h-βTrCP or fragments thereof; or
d) the corresponding mRNA and DNA sequences.

7. Use of the h-βTrCP protein or the peptide fragments according to any one of claims 1 to 5 for the screening of anti-HIV antiviral agents capable of inhibiting the interaction between h-βTrCP protein and Vpu protein.

8. Use of the h-βTrCP protein or the peptide fragments according to any one of claims 1 to 5 for the screening of anti-HIV antiviral agents capable of inhibiting the interaction between h-βTrCP protein and Skp1p protein.

9. Use of the nucleic acid sequences according to claim 6 for the screening of anti-HIV antiviral agents capable of inhibiting the interaction between h-βTrCP protein and Vpu protein.

10. Use of the nucleic acid sequences according to claim 6 for the screening of anti-HIV antiviral agents capable of inhibiting the interaction between h-βTrCP protein and Skp1p protein.

11. Use of the h-βTrCP protein or the peptide fragments according to any one of claims 1 to 5 for the screening of antitumoral agents capable of perturbing the regulation of the cell cycle or protein degradation processes in tumoral human cells by modulating the interaction between h-βTrCP protein and Skp1p protein.

12. Use of the nucleic acid sequences according to claim 6 for the screening of antitumoral agents capable of perturbing the regulation of the cell cycle or protein degradation processes in tumoral human cells by modulating the interaction between h-βTrCP protein and Skp1p protein.

13. Use of the h-βTrCP protein or the peptide fragments according to any one of claims 1 to 5 for the screening of anti-inflammatory agents capable of perturbing activation of the NFκB transcription factor by inhibiting the interaction between h-βTrCP protein and the IκB protein.

14. Use of the nucleic acid sequences according to claim 6 for the screening of anti-inflammatory agents capable of perturbing activation of the NFκB transcription factor by inhibiting the interaction between h-βTrCP protein and the IκB protein.

15. Use of the h-βTrCP protein or the peptide fragments according to any one of claims 1 to 5 for the screening of antitumoral agents capable of reactivating the interaction between h-βTrCP protein and a mutated β-catenin protein in tumoral cells, or between h-βTrCP protein and normal β-catenin in tumoral cells devoid of the APC protein.

16. Use of the nucleic acid sequences according to claim 6 for the screening of antitumoral agents capable of reactivating the interaction between h-βTrCP protein and a mutated β-catenin protein in tumoral cells, or between h-βTrCP protein and normal β-catenin in tumoral cells devoid of the APC protein.

17. Use of the h-βTrCP protein or the peptide fragments according to any one of claims 1 to 5 for the screening of anti-Alzheimer agents capable of reducing the degree of degradation of β-catenin by inhibiting the interaction between h-βTrCP protein and the β-catenin protein.

18. Use of the nucleic acid sequences according to claim 6 for the screening of anti-Alzheimer agents capable of reducing the degree of degradation of β-catenin by inhibiting the interaction between h-βTrCP protein and the β-catenin protein.

19. Use of the h-βTrCP protein or the peptide fragments according to any one of claims 1 to 5 for the detection of β-catenin mutations by yeast two-hybrid screening.

20. Use of the nucleic acid sequences according to claim 6 for the detection of β-catenin mutations by yeast two-hybrid screening.

21. Anti-HIV antiviral agents which consist of the peptide fragments of the h-βTrCP protein according to claim 5, devoid of the F-box.

22. Anti-HIV antiviral agents which consist of the peptide fragments of the h-βTrCP protein according to claim 5, devoid of the WD units.

23. Specific antibodies directed against the h-βTrCP protein or the peptide fragments according to any one of claims 1 to 5.

24. Antisense oligonucleotides which block the transcription or translation of the h-βTrCP protein, according to any one of claims 1 to 4, which hybridise with a nucleic acid sequence according to claim 6.

25. Antitumoral agents which consist of the peptide fragments of the h-βTrCP protein according to claim 5 and which possess the F-box.

26. Antitumoral agents which consist of the peptide fragments of the h-βTrCP protein according to claim 5 and which have conserved both the WD units and the F-box.

27. Anti-inflammatory agents which consist of the peptide fragments of the h-βTrCP protein according to claim 5, devoid of the F-box.

28. Transgenic mice which express a transgene for the h-βTrCP protein according to any one of claims 1 to 4.

29. Transgenic mice in which the βTrCP gene has been invalidated.

30. Expression vector, **characterized in that** it comprises a nucleic acid sequence according to claim 6 and the means necessary for its expression.

31. Microorganisms or host cells transformed by an expression vector according to claim 30.

32. Microorganisms or host cells cotransformed by an expression vector containing the gene coding for the Vpu protein and by an expression vector according to claim 30.

33. Microorganisms or host cells cotransformed by an expression vector containing the gene coding for the Skp1p protein and by an expression vector according to claim 30.

34. Microorganisms or host cells cotransformed by an expression vector containing the gene coding for the IκB protein and by an expression vector according to claim 30.

35. Microorganisms or host cells cotransformed by an expression vector containing the gene coding for the oncogenic β-catenin protein and by an expression vector according to claim 30.

## Patentansprüche

1. Humanes βTrCP-Protein (h-βTrCP) zum Targeting von Proteinen zu den Wegen der Degradation durch das Proteasom, **dadurch gekennzeichnet, daß** es die SEQ ID No. 2 hat, daß es in der Lage ist, mit den Proteinen zu interagieren, die geeignet sind, durch das Proteasom abgebaut zu werden und die das Phosphorylierungsmotiv besitzen, welches die Aminosäuren Asp-Ser-Glu-Xaa-Xaa-Ser umfaßt, worin Xaa eine beliebige natürliche Aminosäure ist und worin die Serin-Reste phosphoryliert sind, und daß es die nachfolgenden WD-Motive besitzt:
- erstes WD-Motiv: Aminosäuren 259 bis 292,
- zweites WD-Motiv: Aminosäuren 304 bis 332,
- drittes WD-Motiv: Aminosäuren 343 bis 372,
- viertes WD-Motiv: Aminosäuren 387 bis 415,
- fünftes WD-Motiv: Aminosäuren 427 bis 455,
- sechstes WD-Motiv: Aminosäuren 467 bis 492,
- siebtes WD-Motiv: Aminosäuren 516 bis 544.

2. Protein nach Anspruch 1, **dadurch gekennzeichnet, daß** es in der Lage ist, mit dem Protein Vpu des Virus HIV-1 oder mit den Zellproteinen IκB oder β-Catenin zu interagieren.

3. Protein nach Anspruch 1, **dadurch gekennzeichnet, daß** es eine F-Box besitzt und daß es in der Lage ist, mit dem Protein Skplp zu interagieren.

4. Protein nach irgendeinem der Ansprüche 1 bis 3, **dadurch gekennzeichnet, daß** die F-Box von den Aminosäuren 147 bis 191 gebildet ist.

5. Peptidfragmente des Proteins nach irgendeinem der Ansprüche 1 bis 4, die aus der Zugabe, der Unterdrückung und/oder dem Ersetzen einer oder mehrerer Aminosäuren resultieren, wobei die genannten Peptidfragmente die Aktivität der Interaktion mit dem Protein Vpu des Virus HIV-1, dem Zellprotein IκB oder dem Zellprotein β-Catenin und/oder mit dem Protein Skplp bewahrt haben.

6. Nukleinsäuresequenzen, die für das humane Protein h-βTrCP und die Peptidfragmente nach irgendeinem der Ansprüche 1 bis 5 codieren, **dadurch gekennzeichnet, daß** sie gebildet sind von:
a) der DNA-Sequenz SEQ ID No. 1 und den Nukleinsäurefragmenten, die für die genannten Peptidfragmente codieren;
b) den DNA-Sequenzen, die unter strengen Bedingungen mit der Sequenz a) hybridisieren;
c) den DNA-Sequenzen, die aufgrund der Degeneration des genetischen Codes aus den obigen Sequenzen a) und b) resultieren und für das humane Protein h-βTrCP oder dessen Fragmente codieren; und
d) den entsprechenden mRNA- und DNA-Sequenzen.

7. Verwendung des Proteins h-βTrCP oder der Peptidfragmente nach irgendeinem der Ansprüche 1 bis 5 für das Screening von antiviralen Wirkstoffen gegen HIV-1, die geeignet sind, die Interaktion zwischen dem Protein h-βTrCP und dem Protein Vpu zu inhibieren.

8. Verwendung des Proteins h-βTrCP oder der Peptidfragmente nach irgendeinem der Ansprüche 1 bis 5 für das Screening von antiviralen Wirkstoffen gegen HIV-1, die geeignet sind, die Interaktion zwischen dem Protein h-βTrCP und dem Protein Skplp zu inhibieren.

9. Verwendung der Nukleinsäuresequenzen nach Anspruch 6 für das Screening von antiviralen Wirkstoffen gegen HIV, die geeignet sind, die Interaktion zwischen dem Protein h-βTrCP und dem Protein Vpu zu inhibieren.

10. Verwendung der Nukleinsäuresequenzen nach Anspruch 6 für das Screening von antiviralen Wirkstoffen gegen HIV, die geeignet sind, die Interaktion zwischen dem Protein h-βTrCP und dem Protein Skplp zu inhibieren.

11. Verwendung des Proteins h-βTrCP oder der Peptidfragmente nach irgendeinem der Ansprüche 1 bis 5 für das Screening antitumoraler Wirkstoffe, die in der Lage sind, die Regulierung des Zellzyklus oder der Prozesse zum Abbau der Proteine in humanen Tumorzellen durch Modulation der Interaktion zwischen dem Protein h-βTrCP und dem Protein Skplp zu stören.

12. Verwendung der Nukleinsäuresequenzen nach Anspruch 6 für das Screening von antitumoralen Wirkstoffen, die in der Lage sind, die Regulierung des Zellzyklus oder der Prozesse zum Abbau der Proteine in humanen Tumorzellen durch Modulation der Interaktion zwischen dem Protein h-βTrCP und dem Protein Skplp zu stören.

13. Verwendung des Proteins h-βTrCP oder der Peptidfragmente nach irgendeinem der Ansprüche 1 bis 5 für das Screening von antiinflammatorischen Wirkstoffen, die in der Lage sind, die Aktivierung des Transkriptionsfaktors NFκB durch Inhibition der Interaktion zwischen dem Protein h-βTrCP und dem Protein IκB zu stören.

14. Verwendung der Nukleinsäuresequenzen nach Anspruch 6 für das Screening von antiinflammatorischen Wirkstoffen, die in der Lage sind, die Aktivierung des Transkriptionsfaktors NFκB durch Inhibition der Interaktion zwischen dem Protein h-βTrCP und dem Protein IκB zu stören.

15. Verwendung des Proteins h-βTrCP oder der Peptidfragmente nach irgendeinem der Ansprüche 1 bis 5 für das Screening von antitumoralen Wirkstoffen, die geeignet sind, die Interaktion zwischen dem Protein h-βTrCP und einem mutierten β-Catenin-Protein von Tumorzellen oder zwischen dem h-βTrCP und dem normalen β-Catenin von Tumorzellen ohne das APC-Protein zu reaktivieren.

16. Verwendung der Nukleinsäuresequenzen nach Anspruch 6 für das Screening von antitumoralen Wirkstoffen, die geeignet sind, die Interaktion zwischen dem Protein h-βTrCP und dem mutierten β-Catenin-Protein von Tumorzellen oder zwischen dem h-βTrCP und dem normalen β-Catenin von Tumorzellen ohne das APC-Protein zu reaktivieren.

17. Verwendung des Proteins h-βTrCP oder der Peptidfragmente nach irgendeinem der Ansprüche 1 bis 5 für das Screening von Wirkstoffen gegen Alzheimer, die geeignet sind, die Abbaurate des β-Catenin durch Inhibition der Interaktion zwischen dem Protein h-βTrCP und dem β-Catenin-Protein zu reduzieren.

18. Verwendung der Nukleinsäuresequenzen nach Anspruch 6 für das Screening von Wirkstoffen gegen Alzheimer, die geeignet sind, die Abbaurate des β-Catenin durch Inhibition der Interaktion zwischen dem Protein h-βTrCP und dem β-Catenin-Protein zu reduzieren.

19. Verwendung des Proteins h-βTrCP oder der Peptidfragmente nach irgendeinem der Ansprüche 1 bis 5 für die Detektion der β-Catenin-Mutationen durch Hefe-Doppelhybrid-Screening.

20. Verwendung der Nukleinsäuresequenzen nach Anspruch 6 für die Detektion der β-Catenin-Mutationen durch Hefe-Doppelhybrid-Screening.

21. Antivirale Wirkstoffe gegen HIV, die von den Peptidfragmenten des Proteins h-βTrCP nach Anspruch 5 ohne die F-Box gebildet sind.

22. Antivirale Wirkstoffe gegen HIV, die von den Peptidfragmenten des Proteins h-βTrCP nach Anspruch 5 ohne die WD-Motive gebildet sind.

23. Spezifische Antikörper, die gegen das Protein h-βTrCP oder die Peptidfragmente gerichtet sind, wie sie in irgendeinem der Ansprüche 1 bis 5 definiert sind.

24. Antisense Oligonukleotide, welche die Transkription oder die Translation des Proteins h-βTrCP nach irgendeinem der Ansprüche 1 bis 4 blockieren und die mit einer Nukleinsäuresequenz nach Anspruch 6 hybridisieren.

25. Antitumorale Wirkstoffe, die von den Peptidfragmenten des Proteins h-βTrCP nach Anspruch 5 gebildet sind, welche die F-Box aufweisen.

26. Antitumorale Wirkstoffe, die von den Peptidfragmenten des Proteins h-βTrCP nach Anspruch 5 gebildet sind, die sowohl die WD-Motive als auch die F-Box bewahrt haben.

27. Antiinflammatorische Wirkstoffe, die von den Peptidfragmenten des Proteins h-βTrCP nach Anspruch 5 ohne die F-Box gebildet sind.

28. Transgene Mäuse, die ein Transgen des Proteins h-βTrCP nach irgendeinem der Ansprüche 1 bis 4 exprimieren.

29. Transgene Mäuse, bei denen das Gen βTrCP ausgeschaltet wurde.

30. Expressionsvektor, **dadurch gekennzeichnet, daß** er eine Nukleinsäuresequenz nach Anspruch 6 sowie die für deren Expression erforderlichen Mittel umfaßt.

31. Mikroorganismen oder Wirtszellen, die durch einen Expressionsvektor nach Anspruch 30 transformiert sind.

32. Mikroorganismen oder Wirtszellen, die durch einen Expressionsvektor, welcher das für das Protein Vpu codierende Gen umfaßt, sowie durch einen Expressionsvektor nach Anspruch 30 cotransformiert sind.

33. Mikroorganismen oder Wirtszellen, die durch einen Expressionsvektor, welcher das für das Protein Skplp codierende Gen umfaßt, sowie durch einen Expressionsvektor nach Anspruch 30 cotransformiert sind.

34. Mikroorganismen oder Wirtszellen, die durch einen Expressionsvektor, welcher das für das Protein IκB codierende Gen umfaßt, sowie durch einen Expressionsvektor nach Anspruch 30 cotransformiert sind.

35. Mikroorganismen oder Wirtszellen, die durch einen Expressionsvektor, welcher das für das onkogene β-Catenin-Protein codierende Gen umfaßt, sowie durch einen Expressionsvektor nach Anspruch 30 cotransformiert sind.


### LISTE DE SEQUENCES

(1) INFORMATIONS GENERALES:
   (i) DEPOSANT:
      (A) NOM: INSTITUT NATIONAL DE LA SANTE ET DE LA RECHERCHE MEDICALE
      (B) RUE: 101, Rue de Tolbiac
      (C) VILLE: PARIS
      (E) PAYS: FRANCE
      (F) CODE POSTAL: 75654 Cédex13
   (ii) TITRE DE L'INVENTION: Protéine humaine βTrCP de ciblage des protéines vers les voies de dégradation par le protéasome
   (iii) NOMBRE DE SEQUENCES: 8
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 2151 paires de bases
      (B) TYPE: nucléotide
      (C) NOMBRE DE BRINS: simple
      (D) CONFIGURATION: linéaire
   (ii) TYPE DE MOLECULE: ADNc
   (ix) CARACTERISTIQUE:
      (A) NOM/CLE: CDS
      (B) EMPLACEMENT:70..1776
   (xi) DESCRIPTION DE LA SEQUENCE: SEQ ID NO: 1:
   TGCGTTGGCT GCGGCCTGGC ACCAAAGGGG CGGCCCCGGC GGAGAGCGGA CCCAGTGGCC      60
   TCGGCGATT ATG GAC CCG GCC GAG GCG GTG CTG CAA GAG AAG GCA CTC        108
             Met Asp Pro Ala Glu Ala Val Leu Gln Glu Lys Ala Leu
              1               5                  10
   AAG TTT ATG AAT TCC TCA GAG AGA GAA GAC TGT AAT AAT GGC GAA CCC      156
   Lys Phe Met Asn Ser Ser Glu Arg Glu Asp Cys Asn Asn Gly Glu Pro
        15                  20                  25
   CCT AGG AAG ATA ATA CCA GAG AAG AAT TCA CTT AGA CAG ACA TAC AAC      204
   Pro Arg Lys Ile Ile Pro Glu Lys Asn Ser Leu Arg Gln Thr Tyr Asn
    30                  35                  40                  45
   AGC TGT GCC AGA CTC TGC TTA AAC CAA GAA ACA GTA TGT TTA GCA AGC      252
   Ser Cys Ala Arg Leu Cys Leu Asn Gln Glu Thr Val Cys Leu Ala Ser
                    50                  55                  60
   ACT GCT ATG AAG ACT GAG AAT TGT GTG GCC AAA ACA AAA CTT GCC AAT      300
   Thr Ala Met Lys Thr Glu Asn Cys Val Ala Lys Thr Lys Leu Ala Asn
                65                  70                  75
   GGC ACT TCC AGT ATG ATT GTG CCC AAG CAA CGG AAA CTC TCA GCA AGC 348
   Gly Thr Ser Ser Met Ile Val Pro Lys Gln Arg Lys Leu Ser Ala Ser
   80 85 90
   TAT GAA AAG GAA AAG GAA CTG TGT GTC AAA TAC TTT GAG CAG TGG TCA 396
   Tyr Glu Lys Glu Lys Glu Leu Cys Val Lys Tyr Phe Glu Gln Trp Ser
   95 100 105
   GAG TCA GAT CAA GTG GAA TTT GTG GAA CAT CTT ATA TCC CAA ATG TGT 444
   Glu Ser Asp Gln Val Glu Phe Val Glu His Leu Ile Ser Gln Met Cys
   110 115 120 125
   CAT TAC CAA CAT GGG CAC ATA AAC TCG TAT CTT AAA CCT ATG TTG CAG 492
   His Tyr Gln His Gly His Ile Asn Ser Tyr Leu Lys Pro Met Leu Gln
   130 135 140
   AGA GAT TTC ATA ACT GCT CTG CCA GCT CGG GGA TTG GAT CAT ATC GCT 540
   Arg Asp Phe Ile Thr Ala Leu Pro Ala Arg Gly Leu Asp His Ile Ala
   145 150 155
   GAG AAC ATT CTG TCA TAC CTG GAT GCC AAA TCA CTA TGT GCT GCT GAA 588
   Glu Asn Ile Leu Ser Tyr Leu Asp Ala Lys Ser Leu Cys Ala Ala Glu
   160 165 170
   CTT GTG TGC AAG GAA TGG TAC CGA GTG ACC TCT GAT GGC ATG CTG TGG 636
   Leu Val Cys Lys Glu Trp Tyr Arg Val Thr Ser Asp Gly Met Leu Trp
   175 180 185
   AAG AAG CTT ATC GAG AGA ATG GTC AGG ACA GAT TCT CTG TGG AGA GGC 684
   Lys Lys Leu Ile Glu Arg Met Val Arg Thr Asp Ser Leu Trp Arg Gly
   190 195 200 205
   CTG GCA GAA CGA AGA GGA TGG GGA CAG TAT TTA TTC AAA AAC AAA CCT 732
   Leu Ala Glu Arg Arg Gly Trp Gly Gln Tyr Leu Phe Lys Asn Lys Pro
   210 215 220
   CCT GAC GGG AAT GCT CCT CCC AAC TCT TTT TAT AGA GCA CTT TAT CCT 780
   Pro Asp Gly Asn Ala Pro Pro Asn Ser Phe Tyr Arg Ala Leu Tyr Pro
   225 230 235
   AAA ATT ATA CAA GAC ATT GAG ACA ATA GAA TCT AAT TGG AGA TGT GGA 828
   Lys Ile Ile Gln Asp Ile Glu Thr Ile Glu Ser Asn Trp Arg Cys Gly
   240 245 250
   AGA CAT AGT TTA CAG AGA ATT CAC TGC CGA AGT GAA ACA AGC AAA GGA 876
   Arg His Ser Leu Gln Arg Ile His Cys Arg Ser Glu Thr Ser Lys Gly
   255 260 265
   GTT TAC TGT TTA CAG TAT GAT GAT CAG AAA ATA GTA AGC GGC CTT CGA 924
   Val Tyr Cys Leu Gln Tyr Asp Asp Gln Lys Ile Val Ser Gly Leu Arg
   270 275 280 285
   GAC AAC ACA ATC AAG ATC TGG GAT AAA AAC ACA TTG GAA TGC AAG CGA 972
   Asp Asn Thr Ile Lys Ile Trp Asp Lys Asn Thr Leu Glu Cys Lys Arg
   290 295 300
   ATT CTC ACA GGC CAT ACA GGT TCA GTC CTC TGT CTC CAG TAT GAT GAG 1020
   Ile Leu Thr Gly His Thr Gly Ser Val Leu Cys Leu Gln Tyr Asp Glu
   305 310 315
   AGA GTG ATC ATA ACA GGA TCA TCG GAT TCC ACG GTC AGA GTG TGG GAT 1068
   Arg Val Ile Ile Thr Gly Ser Ser Asp Ser Thr Val Arg Val Trp Asp
   320 325 330
   GTA AAT ACA GGT GAA ATG CTA AAC ACG TTG ATT CAC CAT TGT GAA GCA      1116
   Val Asn Thr Gly Glu Met Leu Asn Thr Leu Ile His His Cys Glu Ala
       335                 340                 345
   GTT CTG CAC TTG CGT TTC AAT AAT GGC ATG ATG GTG ACC TGC TCC AAA      1164
   Val Leu His Leu Arg Phe Asn Asn Gly Met Met Val Thr Cys Ser Lys
   350                 355                 360                 365
   GAT CGT TCC ATT GCT GTA TGG GAT ATG GCC TCC CCA ACT GAC ATT ACC      1212
   Asp Arg Ser Ile Ala Val Trp Asp Met Ala Ser Pro Thr Asp Ile Thr
                   370                 375                 380
   CTC CGG AGG GTG CTG GTC GGA CAC CGA GCT GCT GTC AAT GTT GTA GAC      1260
   Leu Arg Arg Val Leu Val Gly His Arg Ala Ala Val Asn Val Val Asp
               385                 390                 395
   TTT GAT GAC AAG TAC ATT GTT TCT GCA TCT GGG GAT AGA ACT ATA AAG      1308
   Phe Asp Asp Lys Tyr Ile Val Ser Ala Ser Gly Asp Arg Thr Ile Lys
           400                 405                 410
   GTA TGG AAC ACA AGT ACT TGT GAA TTT GTA AGG ACC TTA AAT GGA CAC      1356
   Val Trp Asn Thr Ser Thr Cys Glu Phe Val Arg Thr Leu Asn Gly His
       415                 420                 425
   AAA CGA GGC ATT GCC TGT TTG CAG TAC AGG GAC AGG CTG GTA GTG AGT      1404
   Lys Arg Gly Ile Ala Cys Leu Gln Tyr Arg Asp Arg Leu Val Val Ser
   430                 435                 440                 445
   GGC TCA TCT GAC AAC ACT ATC AGA TTA TGG GAC ATA GAA TGT GGT GCA      1452
   Gly Ser Ser Asp Asn Thr Ile Arg Leu Trp Asp Ile Glu Cys Gly Ala
                   450                 455                 460
   TGT TTA CGA GTG TTA GAA GGC CAT GAG GAA TTG GTG CGT TGT ATT CGA      1500
   Cys Leu Arg Val Leu Glu Gly His Glu Glu Leu Val Arg Cys Ile Arg
               465                 470                 475
   TTT GAT AAC AAG AGG ATA GTC AGT GGG GCC TAT GAT GGA AAA ATT AAA      1548
   Phe Asp Asn Lys Arg Ile Val Ser Gly Ala Tyr Asp Gly Lys Ile Lys
           480                 485                 490
   GTG TGG GAT CTT GTG GCT GCT TTG GAC CCC CGT GCT CCT GCA GGG ACA      1596
   Val Trp Asp Leu Val Ala Ala Leu Asp Pro Arg Ala Pro Ala Gly Thr
       495                 500                 505
   CTC TGT CTA CGG ACC CTT GTG GAG CAT TCC GGA AGA GTT TTT CGA CTA      1644
   Leu Cys Leu Arg Thr Leu Val Glu His Ser Gly Arg Val Phe Arg Leu
   510                 515                 520                 525
   CAG TTT GAT GAA TTC CAG ATT GTC AGT AGT TCA CAT GAT GAC ACA ATC      1692
   Gln Phe Asp Glu Phe Gln Ile Val Ser Ser Ser His Asp Asp Thr Ile
                   530                 535                 540
   CTC ATC TGG GAC TTC CTA AAT GAT CCA GCT GCC CAA GCT GAA CCC CCC      1740
   Leu Ile Trp Asp Phe Leu Asn Asp Pro Ala Ala Gln Ala Glu Pro Pro
               545                 550                 555
   CGT TCC CCT TCT CGA ACA TAC ACC TAC ATC TCC AGA TAAATAACCA          1786
   Arg Ser Pro Ser Arg Thr Tyr Thr Tyr Ile Ser Arg
           560                 565
   TACACTGACC TCATACTTGC CCAGGACCCA TTAAAGTTGC GGTATTTAAC GTATCTGCCA    1846
   ATACCAGGAT GAGCAACAAC AGTAACAATC AAACTACTGC CCAGTTTCCC TGGACTAGCC    1906
   GAGGAGCAGG GCTTTGAGAC TCCTGTTGGG ACACAGTTGG TCTGCAGTCG GCCCAGGACG 1966
   GTCTACTCAG CACAACTGAC TGCTTCAGTG CTGCTATCAG AAGATGTCTT CTATCAATTG 2026
   TGAATGATTG GAACTTTTAA ACCTCCCCTC CTCTCCTCCT TTCACCTCTG CACCTAGTTT 2086
   TTTCCCATTG GTTCCAGACA AAGGTGACTT ATAAATATAT TTAGTGTTTT GCCAGAAAAA 2146
   AAAAA 2151
(2) INFORMATIONS POUR LA SEQ ID NO: 2:
   (i) CARACTERISTIQUES DE LA SEQUENCE:
      (A) LONGUEUR: 569 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Asp Pro Ala Glu Ala Val Leu Gln Glu Lys Ala Leu Lys Phe Met
    1               5                  10                  15
   Asn Ser Ser Glu Arg Glu Asp Cys Asn Asn Gly Glu Pro Pro Arg Lys
               20                  25                  30
   Ile Ile Pro Glu Lys Asn Ser Leu Arg Gln Thr Tyr Asn Ser Cys Ala
           35                  40                  45
   Arg Leu Cys Leu Asn Gln Glu Thr Val Cys Leu Ala Ser Thr Ala Met
       50                  55                  60
   Lys Thr Glu Asn Cys Val Ala Lys Thr Lys Leu Ala Asn Gly Thr Ser
   65                  70                  75                  80
   Ser Met Ile Val Pro Lys Gln Arg Lys Leu Ser Ala Ser Tyr Glu Lys
                   85                  90                  95
   Glu Lys Glu Leu Cys Val Lys Tyr Phe Glu Gln Trp Ser Glu Ser Asp
               100                 105                 110
   Gln Val Glu Phe Val Glu His Leu Ile Ser Gln Met Cys His Tyr Gln
           115                 120                 125
   His Gly His Ile Asn Ser Tyr Leu Lys Pro Met Leu Gln Arg Asp Phe
       130                 135                 140
   Ile Thr Ala Leu Pro Ala Arg Gly Leu Asp His Ile Ala Glu Asn Ile
   145                 150                 155                 160
   Leu Ser Tyr Leu Asp Ala Lys Ser Leu Cys Ala Ala Glu Leu Val Cys
                   165                 170                 175
   Lys Glu Trp Tyr Arg Val Thr Ser Asp Gly Met Leu Trp Lys Lys Leu
               180                 185                 190
   Ile Glu Arg Met Val Arg Thr Asp Ser Leu Trp Arg Gly Leu Ala Glu
           195                 200                 205
   Arg Arg Gly Trp Gly Gln Tyr Leu Phe Lys Asn Lys Pro Pro Asp Gly
       210                 215                 220
   Asn Ala Pro Pro Asn Ser Phe Tyr Arg Ala Leu Tyr Pro Lys Ile Ile
   225                 230                 235                 240
   Gln Asp Ile Glu Thr Ile Glu Ser Asn Trp Arg Cys Gly Arg His Ser
                   245                 250                 255
   Leu Gln Arg Ile His Cys Arg Ser Glu Thr Ser Lys Gly Val Tyr Cys
               260                 265                 270
   Leu Gln Tyr Asp Asp Gln Lys Ile Val Ser Gly Leu Arg Asp Asn Thr
           275                 280                 285
   Ile Lys Ile Trp Asp Lys Asn Thr Leu Glu Cys Lys Arg Ile Leu Thr
       290                 295                 300
   Gly His Thr Gly Ser Val Leu Cys Leu Gln Tyr Asp Glu Arg Val Ile
   305                 310                 315                 320
   Ile Thr Gly Ser Ser Asp Ser Thr Val Arg Val Trp Asp Val Asn Thr
                   325                 330                 335
   Gly Glu Met Leu Asn Thr Leu Ile His His Cys Glu Ala Val Leu His
               340                 345                 350
   Leu Arg Phe Asn Asn Gly Met Met Val Thr Cys Ser Lys Asp Arg Ser
           355                 360                 365
   Ile Ala Val Trp Asp Met Ala Ser Pro Thr Asp Ile Thr Leu Arg Arg
       370                 375                 380
   Val Leu Val Gly His Arg Ala Ala Val Asn Val Val Asp Phe Asp Asp
   385                 390                 395                 400
   Lys Tyr Ile Val Ser Ala Ser Gly Asp Arg Thr Ile Lys Val Trp Asn
                   405                 410                 415
   Thr Ser Thr Cys Glu Phe Val Arg Thr Leu Asn Gly His Lys Arg Gly
               420                 425                 430
   Ile Ala Cys Leu Gln Tyr Arg Asp Arg Leu Val Val Ser Gly Ser Ser
           435                 440                 445
   Asp Asn Thr Ile Arg Leu Trp Asp Ile Glu Cys Gly Ala Cys Leu Arg
       450                 455                 460
   Val Leu Glu Gly His Glu Glu Leu Val Arg Cys Ile Arg Phe Asp Asn
   465                 470                 475                 480
   Lys Arg Ile Val Ser Gly Ala Tyr Asp Gly Lys Ile Lys Val Trp Asp
                   485                 490                 495
   Leu Val Ala Ala Leu Asp Pro Arg Ala Pro Ala Gly Thr Leu Cys Leu
               500                 505                 510
   Arg Thr Leu Val Glu His Ser Gly Arg Val Phe Arg Leu Gln Phe Asp
           515                 520                 525
   Glu Phe Gln Ile Val Ser Ser Ser His Asp Asp Thr Ile Leu Ile Trp
       530                 535                 540
   Asp Phe Leu Asn Asp Pro Ala Ala Gln Ala Glu Pro Pro Arg Ser Pro
   545                 550                 555                 560
   Ser Arg Thr Tyr Thr Tyr Ile Ser Arg
                   565
(2) INFORMATIONS POUR LA SEQ ID NO: 3:
   (i) CARACTERISTIQUES DE LA SEQUENCE:
      (A) LONGUEUR: 19 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 3:
      CCAAACTGCG TATAACGCG 19
(2) INFORMATIONS POUR LA SEQ ID NO: 4:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 4:
      GGTGAATCAA CGTGTTTAGC 20
(2) INFORMATIONS POUR LA SEQ ID NO: 5:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 5:
      GGATGATGTA TATAACTATC 20
(2) INFORMATIONS POUR LA SEQ ID NO: 6:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 6:
      TTTATCCCAG ATCTTGATTG TGTTG 25
(2) INFORMATIONS POUR LA SEQ ID NO: 7:
   (i) CARACTERISTIQUES DE LA SEQUENCE:
      (A) LONGUEUR: 30 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 7:
      CCAGGATCCT TATACAACAT TGACAGCAGC 30
(2) INFORMATIONS POUR LA SEQ ID NO: 8:
   (i) CARACTERISTIQUES DE LA SEQUENCE:
      (A) LONGUEUR: 29 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 8 :
      CCAGGATCCT TAGTCCCAGA TGAGGATTG 29